# EUROPEAN PATENT SPECIFICATION

(11) **EP 1 809 271 B1**
(45) Date of publication and mention of the grant of the patent: **16.06.2010**
(21) Application number: 05769799.7
(22) Date of filing: 28.07.2005
(51) Int. Cl.: A61K 31/165, A61K 31/00, A61K 45/06, A61P 29/00, A61P 25/06, A61P 25/02, A61P 9/00, A61P 13/00, A61P 3/00, A61P 1/00, A61P 17/00, A61P 11/00, A61P 37/00, A61P 25/08, A61P 5/00

(54) **USE OF (R)-(HALOBENZYLOXY) BENZYLAMINO-PROPANAMIDES AS SODIUM AND/OR CALCIUM CHANNEL SELECTIVE MODULATORS**
VERWENDUNG VON (R)-(HALOGENBENZYLOXY)-BENZYLAMINO-PROPANAMIDEN ALS NATRIUM- UND/ODER CALCIUMKANAL-SELEKTIVE MODULATOREN
UTILISATION DE (R)- (HALOBENZYLOXY)BENZYLAMINOPROPANAMIDES COMME MÉDICAMENT AYANT UNE ACTIVITÉ MODULATRICE SÉLECTIVE DES CANAUX SODIQUES ET/OU CALCIQUES

(30) Priority: 10.09.2004 EP 04021525
(43) Date of publication of application: 25.07.2007
(73) Proprietor: Newron Pharmaceuticals S.p.A., 20091 Bresso (MI) (IT)
(72) Inventor: BARBANTI, Elena, I-20093 Cologno Monzese (IT); THALER, Florian, I-39012 Merano (IT); CACCIA, Carla, I-21020 Cardano Al Campo (IT); FARIELLO, Ruggero, I-21016 Luino (IT); SALVATI, Patricia, I-20020 Arese (IT)
(74) Representative: Sgarbi, Renato
(86) International application number: PCT/EP2005/008200
(87) International publication number: WO 2006/027052

(56) References cited:
- EP-A- 0 400 495
- EP-A- 1 438 956
- EP-A- 1 535 908
- EP-B- 1 045 830
- WO-A-99/55322
- WO-A-03/057219
- WO-A-2004/066987
- WO-A-2004/066990
- WO-A-2005/018627
- WO-A-2005/070405
- US-A- 6 011 035
- US-A1- 2002 016 464
- US-B1- 6 420 383
- VENERONI O ET AL: "NW - 1029: A POTENT Na+!+ CHANNEL BLOCKER WITH ANTIHYPERALGESIC EFFECT IN ANIMAL MODELS OF INFLAMMATORY AND NEUROPATHIC PAIN." SOCIETY FOR NEUROSCIENCE ABSTRACT VIEWER AND ITINERARY PLANNER, vol. 2002, 2002, pages Abstract No. 454.3 URL-http://sf, XP008042617 & 32ND ANNUAL MEETING OF THE SOCIETY FOR NEUROSCIENCE; ORLANDO, FLORIDA, USA; NOVEMBER 02-07, 2002
- SABIDO-DAVID CIBELE ET AL: "The therapeutic potential of Na+ and Ca2+ channel blockers in pain management." EXPERT OPINION ON INVESTIGATIONAL DRUGS. OCT 2004, vol. 13, no. 10, October 2004 (2004-10), pages 1249-1261, XP008042607 ISSN: 1744-7658

## Description

The present invention relates to the use of (R)-2-[4-(2-fluorobenzyloxy)benzylamino]propanamide without (S)-2-[4-(2-fluorobenzyloxy)benzylamino]propanamide or (R)-2-[4-(2-chlorobenzyloxy)benzylamino]-N-methylpropanamide without (S)-2-[4-(2-chlorobenzyloxy)benzylamino]-N-methylpropanamide, or the pharmaceutically acceptable salts thereof, for the manufacture of a medicament selectively active as sodium and/or calcium channel modulator useful in preventing, alleviating and curing a wide range of affections where said mechanism(s) play(s) a pathological role, including pain, migraine, inflammatory, urogenital, and gastrointestinal diseases, characterized in that said medicament is substantially free from any MAO inhibitory effect or exhibits a significantly reduced MAO inhibitory effect at dosages that are therapeutically effective in preventing, alleviating and/or curing said affections.

A further aspect of the invention relates to a compound selected from a method of selective treatment of the above said affections the above listed (R)-(halobenzyloxy)benzylamino-propanamides without the respective above listed (S)-halobenzyloxybenzylamino-propanamides for use in wherein the therapeutic activity of said compounds is substantially free from any MAO inhibitory side effect or exhibits significantly reduced MAO inhibitory side effect.

### BACKGROUND OF THE INVENTION

### Chemical background

Substituted benzylaminopropionamide derivatives active on the central nervous system and useful as anti-epileptic, anti-Parkinson, neuroprotective, antidepressant, antispastic and hypnotic agents are disclosed in International Patent Applications Publ. No. WO90/14334, WO94/22808, WO97/05102, and WO 97/05111 (*see also* Pevarello P. et al. "Synthesis and anticonvulsant activity of a new class of 2-[(arylalkyl)amino]alkanamide derivatives", J. Med. Chemistry, 1998, 41, 579-590). Moreover, International Patent Applications Publ. No. WO 99/26614, WO99/35123 and WO99/35125 disclose substituted alpha aminoamide derivatives active on the central nervous system and useful as analgesic agents.

WO 03/020273 discloses pharmaceutical compositions comprising selected α-aminoamide derivatives and gabapentin, pregabalin or tiagabine.

WO 04/062655 discloses the use of certain α-aminoamide derivatives for the manufacture of medicaments for the treatment of head pain conditions.

WO 05/018627 discloses α-aminoamide derivatives as antiinflammatory agents.

WO 04/066987 discloses method of treating gastrointestinal tract disorders using sodium channel modulators.

WO 04/066990 discloses method of treating lower urinary tract disorders using sodium channel modulators.

PCT/EP/2005/000514 discloses α-aminoamide derivatives useful in the treatment of lower urinary tract disorders.

### Biological background

It is well known that sodium channels play an important role in the neuronal network by transmitting electrical impulses rapidly throughout cells and cell networks, thereby coordinating higher processes ranging from locomotion to cognition. These channels are large transmembrane proteins, which are able to switch between different states to enable selective permeability for sodium ions. For this process an action potential is needed to depolarize the membrane, and hence these channels are voltage-gated. In the past few years a much better understanding of sodium channels and drugs interacting with them has been developed.

It has become clear that a number of drugs having an unknown mechanism of action actually act by modulating sodium channel conductance, including local anesthetics, class I antiarrhythmics and anticonvulsants. Neuronal sodium channel blockers have found application with their use in the treatment of epilepsy (phenytoin and carbamazepine), bipolar disorder (carbamazepine, lamotrigine), preventing neurodegeneration, and in reducing neuropathic pain. Various anti-epileptic drugs that stabilize neuronal excitability are effective in neuropathic pain (gabapentin, pregabalin).

In addition, an increase in sodium channel expression or activity has been observed in several models of inflammatory pain, suggesting a role of sodium channels in inflammatory pain.

Calcium channels are membrane-spanning, multi-subunit proteins that allow entry of calcium ions into cells from the extracellular fluid. Commonly, calcium channels are voltage dependent and are referred to as voltage sensitive calcium channels (VSCC). VSCCs are found throughout the mammalian nervous system, where they regulate such varied activities as cellular excitability, transmitter release, intracellular metabolism, neurosecretory activity and gene expression. All "excitable" cells in animals, such as neurons of the central nervous system (CNS), peripheral nerve cells, and muscle cells, including those of skeletal muscles, cardiac muscles and venous and arterial smooth muscles, have voltage dependent calcium channels. Calcium channels have a central role in regulating intracellular calcium ions levels that are important for cell viability and function. Intracellular calcium ion concentrations are implicated in a number of vital processes in animals, such as neurotransmitter release, activation of second messengers and signal transduction systems, muscle contraction, pacemaker activity, and secretion of hormones. It is believed that calcium channels are relevant in certain disease states. A number of compounds useful in treating various cardiovascular diseases in mammals, including humans, are thought to exert their beneficial effects by modulating functions of voltage dependant calcium channels present in cardiac and/or vascular smooth muscle. Compounds with activity against calcium channels have also been used for the treatment of pain. In particular, N-type calcium channels (Cav2.2), responsible for the regulation of neurotransmitters, are thought to play a significant role in nociceptive transmission, as shown in several pharmacological studies. This hypothesis has been validated in the clinic by Ziconotide, a peptide derived from the venom of the marine snail, Conus Magus. A limitation in the therapeutic use of this peptide is that it has to be administered intrathecally in humans (Bowersox S. S. and Luther R. Toxicon, 1998, 36, 11, 1651-1658).

All together these findings indicate that compounds with sodium and/or calcium channel blockade have a high therapeutic potential in preventing, alleviating and curing a wide range of pathologies, including pain, migraine, cardiovascular, urogenital, metabolic and gastrointestinal diseases, where the above mechanisms have been described as playing a pathological role.

Many papers and patents describe sodium channel and/or calcium channel modulators or antagonists for treating and/or modulating a plethora of disorders. Several local anesthetics, antiarrhythmics, antiemetics, antihypertensive, mood stabilizers, agents for the treatment of unipolar depression, cardiovascular diseases, urinary incontinence, diarrhea, inflammation, stroke, epilepsy, neurodegenerative conditions, nerve cell death, anticonvulsants, neuropathic pain, migraine, acute hyperalgesia and inflammation, renal disease, allergy, asthma, bronchospasm, dysmenorrhea, esophageal spasm, glaucoma, urinary tract disorders, gastrointestinal motility disorders, premature labour, obesity are able to modulate these channels.

A selection of references is reported below: C. Alzheimer describes in Adv. Exp. Med. Biol. 2002, 513, 161-181, sodium and calcium channels as targets of neuroprotective substances.

Vanegas and Schaible (Pain 2000, 85, 9-18) discuss effects of antagonists of calcium channels upon spinal mechanisms of pain, hyperalgesia and allodynia.

U.S. Patent 5,051,403 relates to a method of reducing neuronal damage associated with an ischemic condition, such as stroke, by administration of binding/inhibitory omega-conotoxin peptide wherein the peptide is characterized by specific inhibition of voltage-gated calcium channel currents selectively in neuronal tissues.

U.S. Patent 5,587,454 relates to compositions and methods of producing analgesia particularly in the treatment of pain and neuropathic pain.

U.S. Patent 5,863,952 relates to calcium channel antagonists for the treatment of ischaemic stroke.

U.S. Patent 6,011,035 relates to calcium channel blockers, useful in the treatment of conditions such as stroke and pain.

U.S. Patent 6,117,841 relates to calcium channel blockers and their use in the treatment of stroke, cerebral ischemia, pain, head trauma or epilepsy.

U.S. Patent 6,362,174 relates to N-type calcium channel blockers in the treatment of stroke, cerebral ischemia, pain, epilepsy, and head trauma.

U.S. Patent 6,380,198 concerns the use of the calcium channel blocker flunarizine for the topical treatment of glaucoma.

U.S. Patent 6,420,383 and U.S. Patent 6,472,530 relate to novel calcium channel blockers, useful for treating and preventing a number of disorders such as hypersensitivity, allergy, asthma, bronchospasm, dysmenorrhea, esophageal spasm, glaucoma, premature labor, urinary tract disorders, gastrointestinal motility disorders and cardiovascular disorders.

U.S. Patent 6, 458, 781 relates to compounds that act to block calcium channels and their use to treat stroke, cerebral ischemia, pain, head trauma or epilepsy.

U.S. Patent 6,521,647 relates to the the use of calcium channel blockers in the treatment of renal disease in animals, especially chronic renal failure.

WO 97/10210 relates to tricyclic heterocyclic derivatives, and their use in therapy, in particular as calcium channel antagonists, e.g. for the treatment of ischaemia, in particular ischaemic stroke.

WO 03/018561 relates to quinoline compounds as N-type calcium channel antagonists and methods of using such compounds for the treatment or prevention of pain or nociception.

WO 03/057219 relates to sodium channel blockers useful as agents for treating or modulating a central nervous system disorder, such as neuropathic pain, inflammatory pain, inflammation-related pain or epilepsy.

Monoamine oxidase (MAO) is an enzyme present in the outer mitocondrial membrane of neuronal and non-neuronal cells. Two isoforms of MAO exist: MAO-A and MAO-B. MAO enzymes are responsible for the oxidative deamination of endogenous and xenobiotic amines, and have a different substrate preference, inhibitor specificity, and tissue distribution. For MAO-A serotonin, noradrenaline and adrenaline are preferential substrates, and clorgyline is a selective MAO-A inhibitor; whereas MAO-B prefers β-phenylethylamine as a substrate, and is selectively inhibited by selegiline. Dopamine, tyramine and tryptamine are oxidized by both MAO-A and MAO-B, in particular in human brain dopamine is deaminated by 80% by MAO-B.

MAO inhibition allows endogenous and exogenous substrates to accumulate and may thereby, when almost fully inhibited (>90%), alter the dynamics of regular monoamine transmitters. MAO regulate the concentrations in the brain of the most important neurotransmitters such as noradrenaline, serotonin and dopamine which are related to emotion, anxiety and movement. Thus, MAO activity is thought to be closely linked to various psychiatric and neurological disorders such as depression, anxiety and Parkinson's disease (PD) and aging in general.

MAO-A inhibitors are mainly used in psychiatry for the treatment of major, refractory and atypical depression as a consequence of their ability to increase the reduced serotonin and noradrenalin brain levels. More recently, MAO-A inhibitors have been used to treat patients with anxiety disorders such as social phobia, panic disorders, post-traumatic stress disorders and obsessive compulsive disorders.

MAO-B inhibitors are mainly used in neurology for the treatment of PD.

There is also recent evidence and interest in the role of MAO-B in other pathological conditions such as Alzheimer disease (AD). So far no evidence have been reported on MAO-B involvement in the metabolism of co-transmitters, such as colecystokinin, substance P, somatostatin and neurotensin, which are involved in the modulation of pain sensation. For this reason there is no scientific rationale for the use of MAO-B inhibitors in pain syndromes.

Adverse drug reactions during clinical practice with MAO inhibitors have been reported. First generation of non-selective and irreversible MAO inhibitors, such as tranylcypromide and phenelzine, have serious side effects, including hepatotoxicity, orthostatic hypotension and most importantly hypertensive crisis that occurs following the ingestion of foods containing tyramine (Cooper AJ.-Tyramine and irreversible monoamine oxidase inhibitors in clinical pratice.- Br J Psych Suppl 1989:38-45).

When these non-selective and irreversible MAO inhibitors are used, a strict tyramine-reduced diet must be observed. The pressor sensitivity towards tyramine is normalized 4 weeks after cessation of tranylcypromide therapy and more than 11 weeks after cessation of phenelzine therapy.

Selegiline, a selective and irreversible MAO-B inhibitor, especially when used in combination with levodopa in patients with PD, can cause anorexia/nausea, dry mouth, dyskinesia and orthostatic hypotension, the latter being most problematic (Volz H.P. and Gleiter C.H.- Monoamine oxidase inhibitors. A perspective on their use in the elderly.- Drugs Aging 13 (1998), pp. 341-355).

In monotherapy, anorexia/nausea, musculoskeletal injuries, and cardiac arrhytmias occurred more often in patients receiving selegiline compared with those receiving placebo. Apart from these adverse effects, increased rates of elevated serum AST and ALT levels were noted.

The most frequently reported adverse effect of moclobemide, a selective and reversible MAO-A inhibitor, are sleep disturbances, increased anxiety, restlessness, and headache.

The combination of selective serotonin reuptake inhibitors (SSRIs) and moclobemide has good efficacy in cases of refractory depression, but has created controversy as to whether toxic side effects, such as serotonergic syndrome, result from this combination (Baumann P.-Pharmacokinetic-pharmacodynamic relationship of the selective serotonin reuptake inhibitors. Clin Pharmacokinet 31 (1996), pp 444-469). Because of cardiac arrhythmias and increased liver enzyme levels, electrocardiogram and laboratory values should be checked regularly.

Many types of physiologic changes that occur with aging affect the pharmacodynamics and pharmacokinetics of MAO inhibitors. Indeed, pharmacokinetic variables in the elderly are markedly different form those in younger patients. These variables including absorption, distribution, metabolism and excretion have to be taken into account to avoid or minimize certain adverse effects and drug-drug interactions. Elderly patients are generally more susceptible to side effects, including adverse drug reactions. Hypertensive crisis may occur more frequently in elderly, because the cardiovascular system of the elderly is compromised by age.

The use of sympathomimetic drugs in combination with MAO inhibitors may also elevate blood pressure. In addition, compared with placebo, phenelzine was associated with a significantly higher incidence of drowsiness, tremor, dyskinesia, diarrhea, micturition difficulties, orthostatic effects, and adverse dermatological effects. It is interesting to note that in the elderly, headache is reported with a higher frequency during treatment with moclobemide (Volz H.P. and Gleiter C.H.- Monoamine oxidase inhibitors. A perspective on their use in the elderly. Drugs Aging 13 (1998), pp. 341-355).

MAO inhibitors are sometimes prescribed for depression. Because of the potential risk of suicide, adverse drug reactions and toxicity due to overdose are important factors to consider when choosing an antidepressant. In addition, when MAO inhibitors are used in high dosage, adverse cardiovascular effects seem to increase considerably; and because, with most available drugs, MAO selectivity is lost with such high doses, tyramine can induce potentially dangerous hypertensive reactions. Acute overdose with MAO inhibitors causes agitation, hallucinations, hyperpyrexia, hyperreflexia and convulsions. Abnormal blood pressure is also a toxic sign, so that gastric lavage and maintenance of cardiopulmonary function may be required.. Overdose of traditional non-selective and irreversible MAO inhibitors is considerably dangerous and sometimes fatal (Yamada and Richelson, 1996. Pharmacology of antidepressants in the elderly. In: David JR, Snyder L., editors. Handbook of pharmacology of aging. Boca Raton: CRC Press 1996).

In the treatment of the affections wherein sodium and calcium channels mechanism(s) play(s) a pathological role and, in particular, of pain syndromes (either of neuropathic or inflammatory type) inhibition of MAO enzymes is of no benefits. The most clinically active anti-nociceptive drugs are devoid of MAO inhibition. On the contrary, MAO inhibitory side effects may impose at least two types of negative limitations.
1) Dietary: eating food with high tyramine content may cause severe, even life threatening increase of systemic blood pressure (the so called "cheese-effect").
2) Pharmacological: pain is often treated with a combination of drugs such as opioid derivatives and tricyclic antidepressant. With MAO inhibitors such association is dangerous as it may cause the serotoninergic syndrome (agitation, tremors, hallucination, hyperthermia and arrhythmias).

Thus, eliminating or significantly reducing MAO inhibitory activity in medicaments active as sodium and/or calcium channel modulators useful in preventing, alleviating and curing a wide range of pathologies where said mechanism(s) play(s) a pathological role, (such as pain, migraine, cardiovascular, inflammatory, urogenital, metabolic and gastrointestinal diseases) is an unexpected and substantial therapeutic improvement versus compounds of similar efficacy but with the above mentioned side effects. Said improvement is particularly desirable for the medicaments active as sodium and/or calcium channel modulators useful, in particular, for the treatment of pain syndromes.

Taken into account these findings on MAO inhibitors and, in particular, lacking any evidence for a MAO-B role in pathological affections like pain, migraine, cardiovascular, inflammatory, urogenital, metabolic and gastrointestinal diseases, compounds indicated in these above conditions should not possess MAO-B inhibitory activity, which if present may increment undesired adverse events.

Medicaments which are "selectively active as sodium and/or calcium modulators" or a useful for the "selective treatment" of phatological affections, disorders or diseases wherein the sodium and/or calcium channel mechanism(s) play(s) a pathological role should be preferred. With this expression are intended medicaments which, when administered to a patient in need thereof in amounts that are effective in the treatment of the above said affections wherein the above said mechanism(s) play(s) pathological role, do not exhibit any MAO inhibitory activity or exhibit a significantly reduced MAO inhibitory activity, thus resulting in avoidance of side effects due to accumulation of endogenous and exogenous monoamine transmitters.

It is a primary object of this invention the use of selected (halobenzyloxy)-benzylamino-propanamides for the manufacture of medicaments active as sodium and/or calcium channel modulators for the treatment of pathologies where the above said mechanism(s) play(s) a pathological role, said medicaments being substantially free from any MAO-B inhibitory activity or having significantly reduced MAO inhibitory activity and, therefore, having a reduced potential for unwanted side effects. Said use provides an improved selective resource for the prevention, alleviation and/or cure of the above said pathological affections.

### DESCRIPTION OF THE INTENTION

The object of the present invention relates to the use of (R)-2-[4-(2-fluorobenzyloxy)benzylamino]propanamide without (S)-2-[4-(2-fluorobenzyloxy)benzylamino]propanamide or (R)-2-[4-(2-chlorobenzyloxy)benzylamino]-N-methylpropanamide without (S)-2-[4-(2-chlorobenzyloxy)benzylamino]-N-methylpropanamide, or a pharmaceutically acceptable salt thereof for the manufacture of a medicament selectively active as sodium and/or calcium channel modulator useful in preventing, alleviating and/or curing affections where said mechanism(s) play(s) a pathological role as defined in the claims, characterized in that said medicament is substantially free from any MAO inhibitory effect or exhibits a significantly reduced MAO inhibitory effect at dosages that are therapeutically effective in preventing, alleviating and/or curing said affections.

Said diseases include pain, migraine, inflammatory, urogenital, and gastrointestinal diseases. According to a further aspect of this invention, the affections that can be prevented, alleviated or cured with the above mentioned compounds and the pharmaceutically acceptable salts thereof preferably consist in pain syndromes (either of neuropathic and/or inflammatory type) and/or migraine, and/or urogenital and/or gastrointestinal diseases as defined in the claims.

These compounds have sodium and/or calcium channel modulating activity with an unexpected.selectivity profile when compared to other derivatives of the same chemical class which are active as sodium and/or calcium channel modulators and, in particular, when compared to the corresponding S-isomers. Indeed, it has been shown, through predictive pharmacological tests, that the ratio between the doses of the invention compounds active as sodium and/or calcium channel modulators and the doses of the same products active as MAO-B enzyme inhibitors decreases in an unexpected and significant manner.

In this description and claims, the expression "sodium and/or calcium channel modulator(s)" means compounds able to block sodium and/or calcium currents in a voltage dependent manner.

The compound (R)-2-[4-(2-fluorobenzyloxy)benzylamino]propanamide is mentioned as a single isomer or racemate mixture in EP 1045830 B1 (and WO 99/35125) without any specific information about its preparation and characterization.

The compound (R)-2-[4-(2-chlorobenzyloxy)benzylamino]-N-methylpropanamide is disclosed in the form of the respective salt with methanesulfonic acid in EP 0400495 B1 (and WO 90/14334).

It is also an object of this invention to provide a therapeutically effective amount of (R)-2-[4-(2-fluorobenzyloxy)benzylamino]propanamide without (S)-2-[4-(2-fluorobenzyloxy)benzylamino]propanamide or (R)-2-[4-(2-chlorobenzyloxy)benzylamino]-N-methylpropanamide without (S)-2-[4-(2-chlorobenzyloxy)benzylamino]-N-methylpropanamide, or a pharmaceutically acceptable salts thereof, for use in a method for selectively preventing, alleviating and/or curing pathological affections where sodium and/or calcium channel mechanism(s) play(s) a pathological role as defined in the claims, wherein the therapeutic activity of said compound is substantially free from any MAO inhibitory side effect or exhibits significantly reduced MAO inhibitory side effect.

According to a further aspect of this invention, the pathological affections where the sodium or calcium channel mechanism(s) play(s) a pathological role include pain, migraine, inflammatory, urogenital, and gastrointestinal diseases as defined in the claims; preferably, said pathological affections comprise pain syndromes either of neuropathic or inflammatory type.

The compounds used in the invention and the salts thereof can be obtained by a process comprising the reaction of compounds of general formula **I** 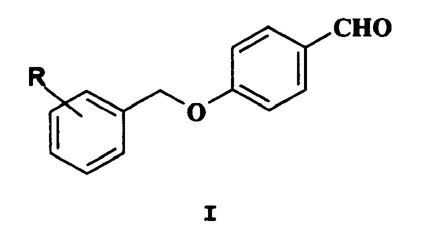 wherein R represents a 2-fluoro or 2-chloro substituent with a compound of formula **II** 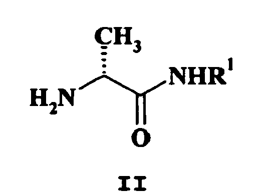 wherein R¹ represents hydrogen, when R in the compound of formula II represents a 2-fluoro substituent, or a methyl group, when R in the compound of formula II represents a 2-chloro substituent.

Compounds **I**, and **II** are commercially available compounds.

The reaction of a compound of formula **I** with the compound of formula **II** to give the corresponding (R)-2-[(halobenzyloxy)benzylamino]-propanamide, is a reductive amination reaction, which can be carried out according to well known methods. Preferably, it may be performed under nitrogen atmosphere, in a suitable organic solvent, such as an alcohol, e.g. a lower alkanol, in particular methanol, or in acetonitrile, or in tetrahydrofuran, at a temperature ranging from about 0°C to about 80°C, in the presence of a reducing agent, the most appropriate being sodium borohydride or sodium cyanoborohydride. Occasionally Titanium IV isopropylate and molecular sieves can be added to the reaction mixture for facilitating the reaction.

### PHARMACOLOGY

The compounds for use in the invention are voltage dependent blockers of the calcium and/or sodium channels as demonstrated by fluorescence calcium influx assay and electrophysiological studies.

The sodium channel modulating activity of the selective (R)-2-[(halobenzyloxy)benzylamino]-propanamides was measured through electrophysiological assays using the two electrodes voltage clamp (TEVC) technique in isolated Xenopus oocytes expressing the Na channel Nav 1.3.

The N-type calcium channel modulating activity of the (R)-2-[(halobenzyloxy)benzylamino]-propanamides was measured through a fluorescence based calcium influx assay.

The MAO-B blocking activity of the above compounds was measured by using an in-vitro enzyme activity assay.

The In-vivo activity of the above compounds as analgesics was assessed through a mice formalin test.

The selectivity of the compounds used in this invention has been evaluated by comparison with other 2-[(halobenzyloxy)benzylamino]-propanamide derivatives that are known to be active as analgesics, according to EP 1045830 B1, in particular, in comparison with the respective (S)-isomers and with both the (R) and (S) isomer of 2-[4-(3-chlorobenzyloxy)benzylamino]propanamide.

These comparison tests show that while the (R)-isomers used in this invention have substantially the same degree of analgesic activity as the comparison compounds, their activity as MAO-B blockers is at least 40-90 fold lower than that of the comparison compounds.

In addition, the ratio between the doses of the R-isomers of this invention, active as Na⁺ and/or Ca⁺ modulators and the doses of the same products active as MAO-B enzyme inhibitors is much lower than that of the comparison compounds, thus providing a further confirmation of their selective profile.

Such substances also exhibit " use-dependency" when the sodium channels are blocked i.e. maximum blockage of the sodium channels is only achieved after repeated stimulation of the sodium channel. Consequently, the substances preferably bind to sodium channels which are multiply activated. As a result the substances are capable of activity preferentially in those regions of the body which are pathologically over-stimulated, as illustrated by patch-clamp experiments which show that the compounds according to the invention block the electrically stimulated sodium channel in a " use-dependent" manner.

As a consequence of these mechanisms the compounds used in the invention are active in vivo when orally administered in the range of 0.1 to 100 mg/kg in the formalin animal model of persistent pain.

In view of the above described mechanisms of action, the compounds used in the present invention are particularly useful in the selective treatment or prevention of neuropathic pain. Neuropathic pain syndromes include diabetic neuropathy; sciatica; nonspecific lower back pain; multiple sclerosis pain; fibromyalgia: HIV-related neuropathy; neuralgia, such as post-herpetic neuralgia and trigeminal neuralgia; and pain resulting from physical trauma, amputation, cancer, toxins or chronic inflammatory conditions; spinal cord, nerve root, peripheral nerve and central pain pathways compressions.

The compounds used in the invention are also useful for the selective treatment of chronic pain. Chronic pain includes chronic pain caused by inflammation, ostheoarthritis, rheumatoid arthritis or as sequela to disease, acute injury or trauma and includes upper back pain or lower back pain (resulting from cyotomatic, regional or primary epine disease (such as radiculopathy), bone pain (due to osteoarthritis, osteoporosis, bone metastasis or unknown reasons), pelvic pain, spinal cord injury-associated pain, cardiac chest pain, non-cardiac chest pain, central post-stroke pain, myofascial pain, cancer pain, AIDS pain, sickle cell pain, geriatric pain or pain caused by headache, temporomandibular joint syndrome, gout, fibrosis or thoracic outlet syndromes, pain related to surgery and sequaelae of surgery.

The compounds used in the invention are also useful in the selective treatment of acute pain caused by acute injury, illness, sports-medicine injuries, carpal tunnel syndrome, burns, musculoskeletal sprains and strains, musculotendinous strain, cervicobrachial pain syndromes, gastric ulcer, duodenal ulcer, dysmenorrhea, endometriosis or surgery such as open heart or bypass surgery, post operative pain, kidney stone pain, gallbladder pain, gallstone pain, obstetric pain or dental pain.

The compounds used in the invention are also useful in the selective treatment of migraine, and others headaches, transformed migraine or evolutive headache, cluster headache, tension headache as well as secondary headache disorders, such as the ones derived from infections, metabolic disorders or other systemic illnesses and other acute headaches, paroxysmal hemicrania resulting from a worsening of the above mentioned primary and secondary headaches.

The compounds used in the invention are useful in the selective treatment of inflammatory processes of the muscular-skeletal system such as ankylosing spondylitis, cervical arthritis, fibromyalgia, gout, juvenile rheumatoid arthritis, lumbosacral arthritis, osteoarthritis, osteoporosis, psoriatic arthritis, rheumatic disease; disorders affecting skin and related tissues: eczema, psoriasis, dermatitis and sunburn; disorders of the respiratory system: asthma, allergic rhinitis and respiratory distress syndrome, lung disorders in which inflammation is involved such as bronchitis; chronic obstructive pulmonary disease; disorders of the immune and endocrinological systems: periarteritis nodosa, thyroiditis, multiple sclerosis, sarcoidosis, Bechet's syndrome, polymyositis, gingivitis.

The compounds used in the invention are also useful in the selective treatment of gastrointestinal (GI) tract disorders such as inflammatory bowel disorders including ulcerative colitis, Crohn's disease, ileitis, proctitis, celiac disease, enteropathies microscopic or collagenous colitis, eosinophilic gastroenteritis, or pouchitis resulting after proctocolectomy and post ileonatal anastomosis, and irritable bowel syndrome including pylorospasm, nervous indigestion, spastic colon, spastic colitis, spastic bowel, intestinal neurosis, functional colitis, mucous colitis and laxative colitis; but also for treatment of atrophic gastritis, gastritis varialoforme, peptic ulceration, pyrosis, damage to the GI tract by *Helicobacter pylori*, gastroesophageal reflux disease, gastroparesis such as diabetic gastroparesis; and other functional bowel disorders such as non-ulcerative dyspepsia (NUD), emesis, diarrhea, and visceral inflammation.

The compounds used in the invention are also useful in the selective treatment of disorders of the genito-urinary tract such as overactive bladder, prostatitis such as chronic bacterial and chronic non-bacterial prostatitis, prostadynia, interstitial cystitis, urinary incontinence, adnexitis, pelvic inflammation, bartholinities and vaginitis.

The compounds used in the invention are also useful for the selective treatment of all other conditions mediated by the inhibition of voltage gated sodium channels and/or voltage gated calcium channels, as defined in the claims.

It will be appreciated that the compounds used in the invention may advantageously be used in conjunction with one or more other therapeutic agents. Examples of suitable agents for adjunctive therapy include a 5HT_{1B/1D} agonist, such as a triptan (e.g. sumatriptan or naratriptan); an adenosine A1 agonist; an EP ligand; an NMDA modulator, such as a glycine antagonist; a substance P antagonist (e.g. an NK1 antagonist); a cannabinoid; acetaminophen or phenacetin; a 5-lipoxygenase inhibitor; a leukotriene receptor antagonist; a DMARD (e.g. methotrexate); gabapentin a tricyclic antidepressant (e.g. amitryptiline); a neurone stabilising antiepileptic drug; a matrix metalloproteinase inhibitor; a nitric oxide synthase (NOS) inhibitor, such as an iNOS or an nNOS inhibitor; an inhibitor of the release, or action, of tumor necrosis factor alpha; an antibody therapy, such as monoclonal antibody therapy, an antiviral agent, such as a nucleoside inhibitor (e.g. lamivudine) or an immune system modulator (e.g. interferon) ; an analgesic, such as a a cyclooxygenase-2. inhibitor; a local anaesthetic; a stimulant, including caffeine; an H2-antagonist (e.g. ranitidine); a proton pump inhibitor (e.g. omeprazole); an antacid (e.g. aluminium or magnesium hydroxide; an antiflatulent (e.g. semethicone); a decongestant (e.g. phenylephrine, phenylpropanolamine, pseudoephedrine, oxymetazoline, epinephrine, naphazoline, xylometazoline, propylhexedrine, or levodesoxyephedrine); antitussive (e.g. codeine, hydrocodone, carmiphen, carbetapentane, or dextramethorphan); a diuretic; or a sedating or non-sedating antihistamine. It is to be understood that the present invention includes the use of a compound according to this invention, or a pharmaceutically acceptable salt thereof in combination with one or more therapeutic agents.

The compounds used in the present invention are useful in human and veterinary medicine. It is to be understood that as used herein the terms "treatment" or "treating" whenever not specifically defined otherwise, include prevention, alleviation and cure of a pathological affection, in particular, they include both treatment of established symptoms and prophylactic treatment.

Accordingly, the expression "therapeutically effective" when referred to an "amount", a "dose" or "dosage" of the (R)-2-[(halobenzyloxy)benzylamino]-propanamides used in this invention is intended as an "amount", a "dose" or "dosage" of any said compounds sufficient for use in both the treatment of the established symptoms and the prophylactic treatment of the above said pathological affections.

According to the use of this invention the above selectively active R-2-[(halobenzyloxy)benzylamino]-propanamides derivatives and their pharmaceutically acceptable salts can be administered as the "active ingredient" of a pharmaceutically acceptable composition which can be prepared by conventional procedures, for instance by mixing the active ingredient with pharmaceutically acceptable, therapeutically inert organic and/or inorganic carrier materials.

The composition comprising the above defined 2-[(halobenzyloxy)benzylamino]-propanamides derivatives can be administered in a variety forms, e.g. orally, in the form of tablets, troches, capsules, sugar or film coated tablets, liquid solutions, emulsions or suspensions; rectally or intravaginally, in the form of suppositories; parenterally, e.g. by intramuscular, subcutaneous or intravenous injection or infusion, locally and transdermally in form of patch and gel and cream.

Suitable pharmaceutically acceptable, therapeutically inert organic and/or inorganic carrier materials useful in the preparation of such composition include, for example, water, gelatin, gum arabic, lactose, starch, cellulose, magnesium stearate, talc, vegetable oils, cyclodextrins and polyalkyleneglycols. The composition comprising the (R)-2-[(halobenzyloxy)benzylamino]-propanamides mentioned above can be sterilized and may contain further well known components, such as, for example, preservatives, stabilizers, wetting or emulsifying agents, e.g. paraffin oil, mannite monooleate, salts to adjust osmotic pressure, and buffers.

For example, the solid oral forms may contain, together with the active ingredient, diluents, e.g. lactose, dextrose, saccharose, cellulose, corn starch or potato starch; lubricants, e.g. silica, talc, stearic acid, magnesium or calcium stearate, and/or polyethylene glycols; binding agents, e.g. starches, arabic gums, gelatin, methylcellulose, carboxymethylcellulose or polyvinyl pyrrolidone; disgregating agents, e.g. a starch, alginic acid, alginates or sodium starch glycolate; effervescing mixtures; dyestuffs; sweeteners; wetting agents such as lecithin, polysorbates, laurylsulphates; and, in general, non-toxic and pharmacologically inactive substances used in pharmaceutical formulations. Said pharmaceutical preparations may be manufactured in known manner, for example, by means of mixing, granulating, tabletting, sugar-coating, or film-coating processes.

The oral formulations comprise sustained release formulations that can be prepared in conventional manner, for instance by applying an enteric coating to tablets and granules.

The liquid dispersion for oral administration may be e.g. syrups, emulsions and suspensions.

The syrups may contain as carrier, for example, saccharose or saccharose with glycerine and/or mannitol and/or sorbitol.

Suspensions and emulsions may contain as a carrier, for example, a natural gum, agar, sodium alginate, pectin, methylcellulose, carboxymethylcellulose, or polyvinyl alcohol. The suspensions or solutions for intramuscular injections may contain, together with the active compound, a pharmaceutically acceptable carrier, e.g. sterile water, olive oil, ethyl oleate, glycols, e.g. propylene glycol, and, if desired, a suitable amount of lidocaine hydrochloride. The solutions for intravenous injections or infusion may contain as carrier, for example, sterile water or, preferably, they may be in the form of sterile, aqueous, isotonic saline solutions.

The suppositories may contain, together with the active ingredient, a pharmaceutically acceptable carrier, e.g. cocoa butter, polyethylene glycol, a polyoxyethylene sorbitan fatty acid ester surfactant or lecithin.

Suitable treatment is given 1, 2 or 3 times daily, depending upon clearance rate. Accordingly, the desired dose may be presented in a single dose or as divided doses administered at appropriate intervals, for example two to four or more sub-doses per day.

The pharmaceutical compositions comprising the selectively active (R)-2-[(halobenzyloxy)benzylamino]-propanamides mentioned above will contain, per dosage unit, e.g., capsule, tablet, powder injection, teaspoonful and suppository from 1 to 2500 mg of the active ingredient, preferably from 5 to 1000 mg, most preferably from 10 to 200 mg of the active ingredient.

Optimal therapeutically effective doses to be administered may be readily determined by those skilled in the art and will vary, basically, with the strength of the preparation, with the mode of administration and with the advancement of the condition or with the type of disorder treated. In addition, factors associated with the particular subject being treated, including subject age, weight, diet and time of administration, will result in the need to adjust the dose to an appropriate therapeutically effective level. In general therapeutically effective daily dosages of the compounds used in the invention in patient in need of the selective treatment of the above mentioned affections wherein the sodium and/or calcium channel mechanism(s) play(s) a pathological role range from 0.05 to 100 mg/kg, preferably from 0.1 to 50 mg/kg, most preferably 0.5 to 10 mg/kg of body weight.

The following Examples further illustrate the invention.

### Example 1

### (R)-2-[4-(2-fluorobenzyloxy)benzylamino]propanamide

To 50 ml of dry methanol with bubbling in (R) alaninamide hydrochloride (1.37 g, 11 mmol), 4-(2-fluorobenzyloxy)benzaldehyde (2.3 g 10 mmol), triethylamine (1,12 g, 11 mmol) and 1g of 3-Å molecular sieves were added and the mixture was stirred for 4 h at 40 °C. The temperature was then lowered to 10 °C and sodium borohydryde (0.19 g, 5 mmol) was added in 15'. The reaction mixture was stirred for 6h at room temperature, then it was filtered and evaporated to dryness under vacuo. The residue was taken up with water and toluene at 60 °C, and the organic phase was washed twice with warm water and dried at the same temperature with anhydrous sodium sulphate. The solution was filtered, and gradually cooled at 10 °C. The precipitate was filtered, washed with a small amount of cooled toluene and dried under vacuum to give 2,69 g (89.0% yield) of white crystals.

### Example 2

### (R)-2-[4-(2-fluorobenzyloxy)benzylamino]propanamide methanesulfonate

To a solution of (R)-2-[4-(2-fluorobenzyloxy)benzylamino]-propanamide (2.5 g, 8.3 mmol) in 40 ml of ethyl acetate, the stoichiometric amount of methanesulfonic acid (0.80 g) diluted in 10 ml of ethyl acetate was added under stirring at room temperature. After 1h the white crystals were filtered, washed with 5 ml of ethyl acetate and dried in a vacuum oven to give 3.26 g (98.8% yield) of the title compound: m.p. 240-241 °C
¹H-NMR (DMSO-d₆) δ: 1.39 (d, J = 6.9 Hz, 3H, C*H*₃CH), 2.30 (s, 3H, CH₃SO₃⁻), 3.71 (q, J = 6.9 Hz, 1H, CH₃C*H*), 4.01 (m, 2H, ArC*H*₂-NH), 5.15 (s, 2H, ArC*H*₂O), 7.08 (m, 2H, H3, H5), 7.1-7.6 (m, 6H, H3', H4', H5', H6', H2, H6), 7.63, 7.89 (2s, 2H, CONH₂), 9.0 (br s, 2H, NH₂⁺); MS m/z 302 (M^{·+}) 258, 230, 215, 109.

Anal. (C₁₇H₁₉FN₂O₂·CH₃SO₃H) C, H, F, N, S.

Analogously were prepared:

### (R)-2-[4-(2-chlorobenzyloxy)benzylamino]-N-methylpropanamide methanesulfonate

¹H-NMR (DMSO-d₆) δ: 1.40 (d, J = 7.0 Hz, 3H, C*H*₃CH), 2.30 (s, 3H, CH₃SO₃⁻), 2, 65 (d, J = 4.5 Hz, 3H, CONHC*H*₃), 3.70 (q, J = 7.0 Hz, 1H, CH₃C*H*), 4.01 (s, 2H, ArC*H*₂-NH), 5.17 (s, 2H, ArC*H*₂O), 7.08 (m, 2H, H3, H5), 7.3-7.7 (m, 6H, H3', H4', H5', H6', H2, H6), 7.63, 7.89 (2s, 2H, CONH₂), 9.0 (br s, 2H, NH₂⁺); MS m/z 332 (M^{·+}) , 274, 246, 231, 125.

Anal. (C₁₈H₂₁ClN₂O₂·CH₃SO₃H) C, H, Cl, N, S.

### (R)-2-[4-(3-chlorobenzyloxy)benzylamino]propanamide methanesulfonate

¹H-NMR (DMSO-d₆) δ: 1.39 (d, J = 6.8 Hz, 3H, C*H*₃CH) , 2.29 (s, 3H, CH₃SO₃⁻), 3.70 (q, J = 6.8 Hz, 1H, CH₃C*H*), 4.01 (s, 2H, ArC*H*₂-NH), 5.15 (s, 2H, ArC*H*₂O), 7.06 (m, 2H, H3, H5), 7.3-7.6 (m, 6H, H2', H4', H5', H6', H2, H6), 7.69-7.91 (2 s, 2H, CONH₂), 8.96 (br s, 2H, NH₂⁺); MS m/z 318 (M^{·+}), 274, 246, 231, 125.

Anal. (C₁₇H₁₉ClN₂O₂-CH₃SO₃H) C, H, Cl, N, S.

### Example 3

### In vitro MAO-B enzyme activity assay

***Membrane preparations (crude mitochondrial fraction) :*** male Wistar rats (Harlan, Italy - 175-200 g) were sacrificed under light anaesthesia and brains were rapidly removed and homogenized in 8 vol. of ice-cold 0.32 M sucrose buffer containing 0.1 M EDTA, pH 7.4. The crude homogenate was centrifuged at 2220 rpm for 10 min and the supernatant recovered. The pellet was homogenized and centrifuged again and the two supernatants were pooled and centrifuged at 9250 rpm for 10 min, +4°C. The pellet was resuspended in fresh buffer and centrifuged at 11250 rpm for 10 min, +4°C. The resulting pellet was stored at -80°C until use.

***In vitro enzyme activity assay***: the enzyme activities were assessed with a radioenzymatic assay using the selective substrate ¹⁴C-phenylethylamine (PEA) for MAO-B.

The mitochondrial pellet (500 µg protein) was resuspended in 0.1M phosphate buffer pH 7.4 and 500 µl was added to 50 µl of the test compound or buffer for 30 min at 37°C (preincubation) then the substrate (50 µl) was added. The incubation was carried out for 10 min at 37°C (¹⁴C-PEA, 0.5 µM).

The reaction was stopped by adding 0.2 ml HCl or perchloric acid. After centrifugation, the deaminated metabolites were extracted with 3 ml of toluene and the radioactive organic phase measured by liquid scintillation spectrometry at 90% efficiency. Radioactivity in the eluate indicates the production of neutral and acidic metabolites formed as a result of MAO-B activity.

The enzymatic activity was expressed as nmoles of substrate transformed/mg protein/min. The activity of MAO-B in the sample was expressed as a percentage of control activity in the absence of inhibitors after subtraction of appropriate blank values.

The drug inhibition curves were obtained from at least eight different concentrations, each in duplicate (10⁻¹⁰ to 10⁻⁵ M) and the IC90 values (the drug concentration inhibiting 90% enzyme activity) with confidence intervals determined using linear regression analysis. (aided-computer program).

To reach a significant increase in neurotransmitter levels, MAO-B enzyme activity has to be blocked at least by 90%. The IC90 values for both the (R)-isomers used in this invention and the comparison compounds are reported in Table I.

### Example 4

### Calcium influx assay

32 human neuroblastoma cells constitutively possess both L and N type channels. Under differentiating conditions, IMR32 preferentially express on the membrane surface N- type calcium channels. The remaining L-type calcium channels were blocked using the selective L type blocker, nifedipine. In these experimental conditions, only N type channels can be detected.

IMR32 cells were differentiated using 1mM dibutyryl-cAMP and 2.5 µM bromodeoxyuridine for 8 days (4 times) in 225 cm² flask, then detached, seeded at 200,000 cells/well on 96 poly-lysine-coated plates and further incubated for 18-24 h in the presence of differentiating buffer before use.

The Ca²⁺ Kit Assay (Molecular Devices), based on a fluorescent calcium indicator 485-535 nm wawelength, was used.

Differentiated cells were incubated with dye loading for 30 min at 37°C then, nifedipine alone (1 µM) or in the presence of ω-conotoxin or test compounds were added for further 15 min.

The fluorescence (485-535nm) was measured before and after (30-40 sec) the automated injection of 100 mM KCl depolarizing solution using a Victor plate reader (Perkin Elmer).

The inhibition curves were calculated from 5 concentrations, each in triplicate, and the IC50 determined using a linear regression analysis.

The activity on M-type Calcium channel, expressed as IC50 of the (R)-isomer used in this invention and the comparison compounds is reported in Table I.

### Example 5

### Electrophysiological assay

Experiments for the determination of the tonic block are carried out on isolated Xenopus oocytes expressing the Na channel Nav 1.3. Currents are recorded using the two electrodes voltage clamp (TEVC) technique

### Oocytes preparation:

The frog (Xenopus Laevis) is anesthesized in a solution with 3-aminobenzoic acid ethyl ester (1 g/l) and, after 25 minutes, it is placed on its back on an "iced-bed". The skin and the others tissues are cut, the ovarian lobes are pulled out and kept in ND96∅Ca²⁺ (NaCl 96mM, KCl 2mM, MgCl₂ 1 mM, Hepes 10mM, pH 7.85 with NaOH)

After the removal of the oocytes, the muscle and the skin are sutured separately.

Ovarian lobes are reduced into clusters of 10/20 oocytes, put in tubes with collagenase solution (1 mg/ml) and kept in movement for about 1 h in an incubator.

At the end of this step, when the oocytes are well separated ones from the others, they are rinsed three times with MD96ØCa²⁺ and three times with NDE (ND96ØCa²⁺ + CaCl₂ 0.9mM, MgCl₂ 0.9 mM, piruvate 2.5 mM, gentamicine 50 mg/l)

The oocytes obtained are at different stages of development. Only cells at stages V or VI are selected for RNA injection subsequent experiments.

The day after the preparation, the oocytes are injected (Drummond Nanoject) with 20 ng Navl.3 cRNA and maintained in NDE.

Starting from 48h after the mRNA injection whole cell currents are recorded using a two-microelectrode voltage clamp automated workstation.

Typical Microelectrodes have a resistance of 0.5 to 1 Mohm and are filled with KCl 3M

Control bath solution containes (mM): NaCl 98, MgCl₂ 1, CaCl₂ 1.8, HEPES 5 (pH 7.6).

Compounds are prepared in stock solutions (20mM) and dissolved to the final concentrations in the external bath solution.

### Currents recording:

The current/voltage (I/V) relationship for the Nav1.3 currents expressed in oocytes was first studied in order to determine the membrane potential evoking the maximal activation. Nav1.3 showed the max activation at 0 mV, that we used as test potential (Vtest) for tonic block studies.

The steady - state inactivation properties of the Nav1.3 currents were then studied in order to determine the membrane potentials for the resting state (Vrest) at which channel availabity is maximal (Imax), and the membrane potential for the half maximal inactivation (V ½) producing half of the max current availability (I ½) respectively. This two voltage conditions were then used for the evaluation of the voltage dependence of the tonic block.

Finally a two-step protocol was used to determine the voltage dependence of the block of Nav1.3: the oocytes were clamped at -80 mV, the currents were activated by a 100 ms step pulse to 0 mV (Vtest) from a 3000 ms preconditioning potential at -80 mV (resting, Imax condition) and -40 mV (depolarized, I ½ condition), respectively.

Current amplitudes in the two conditions were recorded in the absence and in the presence of different concentrations of compound (washout was made in between) in oder to determine the concentration - inhibition curves and IC50 values for the tonic block in the depolarized (half max current availability) conditions.

The activity on Nav. 1.3 sodium channel, expressed as IC50 of the (R) -isomers of this invention and of the comparison compounds is reported in Table I.

### Example 6

### Mice Formalin Test

According to a modified protocol from Rosland et al (1990) mice were injected subcutaneously (s.c.) with 20 µl of 2.7% solution of formalin into the plantar surface of left hindpaw and placed immediately into clear PVC observation cambers (23 x 12 x 13 cm).

The test compound (20 mg/kg) was administered p.o. 15 min before formalin injection in a volume of 10 ml/kg body weight to groups of 10 mice per dose. Control group was treated with vehicle.

Pain behavior was quantified by counting the cumulative licking time (seconds) of the injected paw with formalin. Measurements were taken during the late phase 30-40 min after formalin injection (Tjolsen et al 1992).

The analgesic effect of the compounds was calculated as the % of inhibition of the cumulative licking time respect the control group.

As reported in Table II both (R)-isomers used in this invention showed similar (not statistically different) or better analgesic activity than the comparison compounds.

### Results

**Table I.**

| **COMPOUND NAME (1)** | **MAO-B** **IC90 (µM)** | | **Nav 1.3** **Dep.Curr** **IC50 (µM)** | | **Ca 2+** **N-Type** **IC50 (µM)** | |
|---|---|---|---|---|---|---|
| | **R isomer** | **S isomer** | **R isomer** | **S isomer** | **R isomer** | **S isomer** |
| 2-[4-(2-fluorobenzyloxy)benzylamino]-propanamide | 2500 | 28.4 | 149 | 202 | 29.2 | 23 |
| 2-[4-(2-chlorobenzyloxy)benzylamino]-N-methylpropanamide | 122 | 2.8 | 38 | 210 | 8.4 | 30 |
| 2-[4-(3-chlorobenzyloxy)benzylamino]-propanamide | 32.3 | 1.64 | 39 | 79.0 | 20 | 94.0 |

| | | | | | | |
|---|---|---|---|---|---|---|
| (1) All compounds were employed in the form of salts with methanesulfonic acid. | | | | | | |

**Table II**

| **COMPOUND NAME (20mg/kg) (2)** | **FORMALIN TEST % inibition** | |
|---|---|---|
| | **R isomer** | **S isomer** |
| 2-[4-(2-fluorobenzyloxy)benzylamino]propanamide | 80 | 79 |
| 2-[4-(2-chlorobenzyloxy)benzylamino]-N-methylpropanamide | 49 | 32 |
| 2-[4-(3-chlorobenzyloxy)benzylamino]propanamide | 39 | 45 |

| | | |
|---|---|---|
| (2) All compounds were employed in the form of salts with methanesulfonic acid | | |

According to the terms and criteria used in this application and claims a medicament active as sodium and/or channel modulator is considered as substantially free from any MAO-B inhibitory effect at dosages that are therapeutically effective in preventing, alleviating and/or curing affections where said mechanism (s) play(s) a pathological role when both the ratio between the values of IC50 of Nav 1.3 and of IC90 MAO-B and the ratio between the values of IC50 Ca²⁺N-Type and of IC90 MAO-B are lower than 0.1. Similarly, a medicament active as sodium and/or calcium channel modulator is considered as exhibiting a significantly reduced MAO-B inhibitory effect at dosages that are therapeutically effective in preventing, alleviating, and/or curing affections where said mechanism(s) play(s) a pathological role when both the ratio between the values of IC50 Nav 1.3 and IC90 MAO-B and the ratio between the values of IC50 Ca²⁺ and IC90 MAO-B are lower than 0.5 but at least one of them is not lower than 0.1.

## Claims

1. The use of (R)-2-[4-(2-fluorobenzyloxy)benzylamino]propanamide without (S)-2-[4-(2-fluorobenzyloxy)benzylamino]propanamide or (R)-2-[4-(2-chlorobenzyloxy)benzylamino]-N-methylpropanamide without (S)-2-[4-(2-chlorobenzylozy)benzylamino]-N-methylpropanamide, or a pharmaceutically acceptable salt thereof, for the manufacture of a medicament selectively active as sodium and/or calcium channel modulator for preventing, alleviating and/or curing a pathological affection selected from pain, migraine, ankylosing spondylitis, cervical arthritis, fibromyalgia, gout, juvenile rheumatoid arthritics, lumbosacral arthritis, osteoarthritis, osteoporosis, psoriatic arthritis, rheumatic disease, psoriasis, dermatitis, sunburn, asthma, allergic rhinitis, respiratory distress syndrome, bronchitis, chronic obstructive pulmonary disease, periarteritis nodosa, thyroiditis, multiple sclerosis, sarcoidosis, Bechet's syndrome, polymyositis, gingivitis, ulcerative colitis, Crohn's disease, ileitis, proctitis, celiac disease, enteropathies, microscopic or collagenous colitis, eosinophilic gastroenteritis, pouchitis resulting after proctocolectomy and post ileonatal anastomosis, irritable bowel syndrome including pylorospasm, nervous indigestion, spastic colon, spastic colitis, spastic bowel, intestinal neurosis, functional colitis, mucous colitis and laxative colitis, atrophic gastritis, gastritis varialoforme, peptic ulceration, pyrosis, damage to the GI tract by *Helicobacter pylori*, gastroesophageal reflux disease, gastroparesis such as diabetic gastroparesis, non-ulcerative dyspepsia (NUD), emesis, diarrhea, visceral inflammation, overactive bladder, prostatitis such as chronic bacterial and chronic non-bacterial prostatitis, prostadynia, interstitial cystitis, urinary incontinence, adnexitis, pelvic inflammation, bartholinitis and vaginitis, **characterized in that** said medicament is substantially free from any MAO inhibitory effect or exhibits a significantly reduced MAO inhibitory effect at dosages that are therapeutically effective in preventing, alleviating and/or curing said pathological affection.

2. The use according to claim 1 wherein the pathological affection is selected from a pain syndrome, migraine, ulcerative colitis, Crohn's disease, ileitis, proctitis, celiac disease, enteropathies, microscopic or collagenous colitis, eosinophilic gastroenteritis, pouchitis resulting after proctocolectomy and post ileonatal anastomosis, irritable bowel syndrome including pylorospasm, nervous indigestion, spastic colon, spastic colitis, spastic bowel, intestinal neurosis, functional colitis, mucous colitis and laxative colitis, atrophic gastritis, gastritis varialoforme, peptic ulceration, pyrosis, damage to the GI tract by *Helicobacter pylori,* gastroesophageal reflux disease, gastroparesis such as diabetic gastroparesis, non-ulcerative dyspepsia (NUD), emesis, diarrhea, visceral inflammation, overactive bladder, prostatitis such as chronic bacterial and chronic non-bacterial prostatitis, prostadynia, interstitial cystitis, urinary incontinence, adnexitis, pelvic inflammation, bartholinitis and vaginitis.

3. The use according to any of claims 1 and 2 wherein the pathological affection is a pain syndrome.

4. The use according to claim 3 wherein the pain syndrome is a neuropathic pain syndrome.

5. The use according to claim 4 wherein the neuropathic pain syndrome includes: diabetic neuropathy, sciatica, nonspecific lower back pain, multiple sclerosis pain, fibromyalgia, HIV-related neuropathy, neuralgia such as post-herpetic neuralgia and trigeminal neuralgia, and pain resulting from physical trauma, amputation, cancer, toxins or chronic inflammatory conditions, spinal cord, nerve root, peripheral nerve and central pain pathways compressions.

6. The use according to claim 3 wherein the pain syndrome is a chronic pain.

7. The use according to claim 6 wherein the chronic pain includes: chronic pain caused by inflammation, ostheoarthritis, rheumatoid arthritis or as sequela to disease, acute injury or trauma, upper back pain or lower back pain such as resulting from systematic, regional or primary spine disease, such as radiculopathy, bone pain such as due to osteoarthritis, osteoporosis, bone metastasis or unknown reasons, pelvic pain, spinal cord injury-associated pain, cardiac chest pain, non-cardiac chest pain, central post-stroke pain, myofascial pain, cancer pain, AIDS pain, sickle cell pain, geriatric pain or pain caused by headache, temporomandibular joint syndrome, gout, fibrosis or thoracic outlet syndromes, pain related to surgery and sequaelae of surgery.

8. The use according to claim 3 wherein the pain syndrome is an acute pain.

9. The use according to claim 8 wherein the acute pain includes: acute pain caused by acute injury, illness, sport-medicine injuries, carpal tunnel syndrome, burns, musculoskeletal sprains and strains, musculotendinous strain, cervicobrachial pain syndromes, gastric ulcer, duodenal ulcer, dysmenorrhea, endometriosis or surgery (such as open heart or bypass surgery), post operative pain, kidney stone pain, gallbladder pain, gallstone pain, obstetric pain or dental pain.

10. The use according to claim 3 wherein the pain syndrome is of inflammatory type.

11. The use according to any of claims 1 and 2 wherein the pathological affection is migraine.

12. The use according to claim 11 wherein migraine includes: transformed migraine or evolutive headache, cluster headache, tension headache as well as secondary headache disorders such as the ones derived from infections, metabolic disorders or other systemic illnesses and other acute headaches, paroxysmal hemicrania resulting from a worsening of the above mentioned primary and secondary headaches.

13. The use according to claim 1 wherein the pathological affection is selected from ankylosing spondylitis, cervical arthritis, fibromyalgia, gout, juvenile rheumatoid arthritis, lumbosacral arthritis, osteoarthritis, osteoporosis, psoriatic arthritis and rheumatic disease.

14. The use according to claim 1 wherein the pathological affection is selected from psoriasis, dermatitis and sunburn.

15. The use according to claim 1 wherein the pathological affection is selected from asthma, allergic rhinitis, respiratory distress syndrome, bronchitis and chronic obstructive pulmonary disease.

16. The use according to claim 1 wherein the pathological affection is selected from periarteritis nodosa, thyroiditis, multiple sclerosis, sarcoidosis, Bechet's syndrome, polymyositis and gingivitis.

17. The use according to any of claims 1 and 2 wherein the pathological affection is selected from ulcerative colitis, Crohn's disease, ileitis, proctitis, celiac disease, enteropathies, microscopic or collagenous colitis, eosinophilic gastroenteritis, pouchitis resulting after proctocolectomy and post ileonatal anastomosis, irritable bowel syndrome including pylorospasm, nervous indigestion, spastic colon, spastic colitis, spastic bowel, intestinal neurosis, functional colitis, mucous colitis and laxative colitis, atrophic gastritis, gastritis varialoforme, peptic ulceration, pyrosis, damage to the GI tract by *Helicobacter pylori*, gastroesophageal reflux disease, gastroparesis such as diabetic gastroparesis, non-ulcerative dyspepsia (NUD), emesis, diarrhea and visceral inflammation.

18. The use according to any of claims 1 and 2 wherein the pathological affection is selected from overactive bladder, prostatitis such as chronic bacterial and chronic non-bacterial prostatitis, prostadynia, interstitial cystitis, urinary incontinence, adnexitis, pelvic inflammation, bartholinitis and vaginitis.

19. The use of (R)-2-[4-(2-fluorobenzyloxy)benzylamino]propanamide without (S)-2-[4-(2-fluorobenzyloxy)benzylamino]propanamide, or a pharmaceutically acceptable salt thereof, preferably the salt with methanesulfonic acid, according to any of claims 1 to 18.

20. The use of (R)-2-[4-(2-chlorobenzyloxy)benzylamino]-N-methylpropanamide without (S)-2-[4-(2-chlorobenzyloxy)benzylamino]-N-methylpropanamide, or a pharmaceutically acceptable salt thereof, preferably the salt with methanesulfonic acid, according to any of claims 1 to 18.

21. The use according to any of claims 1 to 19 wherein the MAO enzyme is MAO-B isoform.

22. Use of (R)-2-[4-(2-fluorobenzyloxy)benzylamino]propanamide without (S)-2-[4-(2-fluorobenzyloxy)benzylamino]propanamide or (R)-2-[4-(2-chlorobenzyloxy)benzylamino]-N-methylpropanamide without (S)-2-[4-(2-chlorobenzyloxy)benzylamino]-N-methylpropanamide, or a pharmaceutically acceptable salt thereof, preferably the salt with methanesulfonic acid, for the preparation of a medicament for preventing, alleviating and/or curing a pain syndrome, which at the dosages which are therapeutically effective for the treatment of said pain syndrome is substantially free from any MAO-B inhibitory effect or exhibits a significantly reduced MAO-B inhibitory effect.

23. The use according to any of claims 1 to 22 which includes both treatment of established symptoms and prophylactic treatment.

24. The use according to any of claims 1 to 23 wherein the single (R)-isomer or a pharmaceutically acceptable salt thereof is used in conjunction with one or more other therapeutic agents.

25. The use according to claim 24 wherein the other therapeutic agent is gabapentin.

26. A pharmaceutical composition comprising (R)-2-[4-(2-fluorobenzyloxy)benzylamino]propanamide without (S)-2-[4-(2-fluorobenzyloxy)benzylamino]propanamide, or a pharmaceutically acceptable salt thereof, preferably its methanesulfonate, for use in the selective treatment of a pain syndrome or migraine, wherein the therapeutical activity of said compound is substantially free from any MAO inhibitory side effect or exhibits significantly reduced MAO inhibitory side effect.

27. A pharmaceutical composition as in claim 26 wherein the pain syndrome is either of neuropathic or inflammatory type.

28. A pharmaceutical composition comprising (R)-2-[4-(2-chlorobenzyloxy)benzylamino]-N-methylpropanamide without (S)-2-[4-(2-chlorobenzyloxy)benzylamino]-N-methylpropanamide, or a pharmaceutically acceptable salt thereof, preferably its methanesulfonate, for use in the selective treatment of a pain syndrome or migraine, wherein the therapeutical activity of said compound is substantially free from any MAO inhibitory side effect or exhibits significantly reduced MAO inhibitory side effect.

29. A pharmaceutical composition as in claim 28 wherein the pain syndrome is either of neuropathic or inflammatory type.

30. A compound selected from (R)-2-[4-(2-fluorobenzyloxy)benzylamino]propanamide without (S)-2-[4-(2-fluorobenzyloxy)benzylamino]propanamide and (R)-2-[4-(2-chlorobenzyloxy)benzylamino]-N-methylpropanamide without (S)-2-[4-chlorobenzyloxy)benzylamino]-N-methylpropanamide, or a pharmaceutically acceptable salt thereof, for use as a medicament selectively active as sodium and/or calcium channel modulator for use in preventing, alleviating and/or curing a pathological affection selected from pain, migraine, ankylosing spondylitis, cervical arthritis, fibromyalgia, gout, juvenile rheumatoid arthritis, lumbosacral arthritis, osteoarthritis, osteoporosis, psoriatic arthritis, rheumatic disease, psoriasis, dermatitis, sunburn, asthma, allergic rhinitis, respiratory distress syndrome, bronchitis, chronic obstructive pulmonary disease, periarteritis nodosa, thyroiditis, multiple sclerosis, sarcoidosis, Bechet's syndrome, polymyositis, gingivitis, ulcerative colitis, Crohn's disease, ileitis, proctitis, celiac disease, enteropathies, microscopic or collagenous colitis, eosinophilic gastroenteritis, pouchitis resulting after proctocolectomy and post ileonatal anastomosis, irritable bowel syndrome including pylorospasm, nervous indigestion, spastic colon, spastic colitis, spastic bowel, intestinal neurosis, functional colitis, mucous colitis and laxative colitis, atrophic gastritis, gastritis varialoforme, peptic ulceration, pyrosis, damage to the GI tract by *Helicobacter pylori,* gastroesophageal reflux disease, gastroparesis such as diabetic gastroparesis, non-ulcerative dyspepsia (NUD), emesis, diarrhea, visceral inflammation, overactive bladder, prostatitis such as chronic bacterial and chronic non-bacterial prostatitis, prostadynia, interstitial cystitis, urinary incontinence, adnexitis, pelvic inflammation, bartholinitis and vaginitis; **characterized in that** said medicament is substantially free from any MAO inhibitory effect or exhibits a significantly reduced MAO inhibitory effect at dosages that are therapeutically effective in preventing, alleviating and/or curing said pathological affection.

31. A compound of claim 30 for use according to claim 30 wherein the pathological affection is selected from a pain syndrome, migraine, ulcerative colitis, Crohn's disease, ileitis, proctitis, celiac disease, enteropathies, microscopic or collagenous colitis, eosinophilic gastroenteritis, pouchitis resulting after proctocolectomy and post ileonatal anastomosis, irritable bowel syndrome including pylorospasm, nervous indigestion, spastic colon, spastic colitis, spastic bowel, intestinal neurosis, functional colitis, mucous colitis and laxative colitis, atrophic gastritis, gastritis varialoforme, peptic ulceration, pyrosis, damage to the GI tract by *Helicobacter pylori,* gastroesophageal reflux disease, gastroparesis such as diabetic gastroparesis, non-ulcerative dyspepsia (NUD), emesis, diarrhea, visceral inflammation, overactive bladder, prostatitis such as chronic bacterial and chronic non-bacterial prostatitis), prostadynia, interstitial cystitis, urinary incontinence, adnexitis, pelvic inflammation, bartholinitis and vaginitis.

32. A compound of claim 30 for use according to any of claims 30 and 31 wherein the pathological affection is a pain syndrome.

33. A compound of claim 30 for use according to claim 32 wherein the pain syndrome is a neuropathic pain syndrome.

34. A compound of claim 30 for use according to any of claims 30 and 31 wherein the pathological affection is migraine.

35. A compound of claim 30 for use according to any of claims 30 and 31 wherein the pathological affection is a pain syndrome as defined in any of claims 4 to 10.

36. A compound of claim 30 for use according to any of claims 30 and 31 wherein the pathological affection is migraine as defined in claim 12.

## Patentansprüche

1. Verwendung von (R)-2-[4-(2-Fluorbenzyloxy)benzylamino]-propanamid ohne (S)-2-[4-(2-Fluorbenzyloxy)benzylamino]-propanamid oder (R)-2-[4-(2-Chlorbenzyloxy)benzylamino]-N-methylpropanamid ohne (S)-2-[4-(2-Chlorbenzyloxy)benzylamino]-N-methylpropanamid oder eines pharmazeutisch verträglichen Salzes davon für die Herstellung eines Medikaments, das als Natrium- und/oder Calciumkanalmodulator selektiv aktiv ist zur Prävention, Linderung und/oder Heilung einer pathologischen Affektion, ausgewählt aus Schmerz, Migräne, Spondylarthritis, cervikaler Arthritis, Fibromyalgie, Gicht, juveniler rheumatoider Arthritis, lumbosakraler Arthritis, Osteoarthritis, Osteoporose, psoriatischer Arthritis, rheumatischer Erkrankung, Psoriasis, Dermatitis, Sonnenbrand, Asthma, allergischer Rhinitis, Atemnotsyndrom, Bronchitis, chronisch-obstruktiver Lungenerkrankung, Periarteritis nodosa, Schilddrüsenentzündung, Multipler Sklerose, Sarcoidose, Bechet-Syndrom, Polymyositis, Gingivitis, Colitis ulcerosa, Chron'scher Erkrankung, Ileitis, Proctitis, Zöliakie, Enteropathien, mikroskopischer oder kollagenöser Colitis, eosinophiler Gastroenteritis, Pouchitis, resultierend nach Proktokolektomie und nach ileonataler Anastomose, Reizdarmsyndrom, einschließlich Pylorospasmus, nervöser Verdauungsstörung, irritablem Colon, spastischer Colitis, spastischem Darm, Intestinalneurose, Funktionscolitis, mucomembranöser Colitis und laxierender Colitis, atrophischer Gastritis, Gastritis varialoforme, peptischer Ulceration, Sodbrennen, Schädigung am GI-Trakt durch *Heliobacter pylori,* gastroösophagealer Refluxerkrankung, Gastroparese, zum Beispiel diabetischer Gastroparese, Nicht-Ulcus-Dyspepsie (NUD), Emesis, Diarrhöe, visceraler Entzündung, hyperaktiver Blase, Prostatitis, zum Beispiel chronischer bakterieller und chronischer nichtbakterieller Prostatitis, Prostadynie, interstitieller Cystitis, Harninkontinenz, Adnexitis, Beckenentzündung, Bartholinitis und Vaginitis, **dadurch gekennzeichnet, dass** das Medikament bei Dosierungen, die therapeutisch wirksam sind bei der Prävention, Linderung und/oder Heilung der pathologischen Affektion im Wesentlichen frei von einer MAO inhibierenden Wirkung ist oder eine deutlich reduzierte MAO inhibierende Wirkung hat.

2. Verwendung nach Anspruch 1, wobei die pathologische Affektion ausgewählt ist aus Schmerzsyndrom, Migräne, Colitis ulcerosa, Chron'scher Erkrankung, Ileitis, Proctitis, Zöliakie, Enteropathien, mikroskopischer oder kollagenöser Colitis, eosinophiler Gastroenteritis, Pouchitis, resultierend nach Proktokolektomie und nach ileonataler Anastomose, Reizdarmsyndrom, einschließlich Pylorospasmus, nervöser Verdauungsstörung, irritablem Colon, spastischer Colitis, Reizdarm, intestinaler Neurose, funktioneller Colitis, mucomembranöser Colitis und laxierender Colitis, atrophischer Gastritis, Gastritis varialoforme, peptischer Ulceration, Pyrosis, Schädigung am GI-Trakt durch *Helicobacter pylori,* gastroösophagealer Refluxerkrankung, Gastroparese, zum Beispiel diabetischer Gastroparese, Nicht-Ulcus-Dyspepsie (NUD), Emesis, Diarrhöe, visceraler Entzündung, hyperaktiver Blase, Prostatitis, zum Beispiel chronischer bakterieller und chronischer nichtbakterieller Prostatitis, Prostadynie, interstitieller Cystitis, Harninkontinenz, Adnexitis, Beckenentzündung, Bartholinitis und Vaginitis.

3. Verwendung nach einem der Ansprüche 1 und 2, wobei die pathologische Affektion ein Schmerzsyndrom ist.

4. Verwendung nach Anspruch 3, wobei das Schmerzsyndrom ein neuropathisches Schmerzsyndrom ist.

5. Verwendung nach Anspruch 4, wobei das neuropathische Schmerzsyndrom umfasst: diabetische Neuropathie, Ischiassyndrom, nicht spezifischen Schmerz des Kreuzes, Multiple-Sklerose-Schmerz, Fibromyalgie, HIV-bedingte Neuropathie, Neuralgie, zum Beispiel Post-Herpes-Neuralgie und trigeminale Neuralgie, und Schmerz, resultierend aus physischem Trauma, Amputation, Krebs, Toxinen oder chronischen Entzündungszuständen, Kompressionen der Rückenmarks-Nervenwurzel-, peripheren Nerven- und Zentralnerven-Schmerzwege.

6. Verwendung nach Anspruch 3, wobei das Schmerzsyndrom ein chronischer Schmerz ist.

7. Verwendung nach Anspruch 6, wobei der chronische Schmerz umfasst: chronischen Schmerz, verursacht durch Entzündung, Ostheoarthritis, rheumatoider Arthritis oder Folge einer Erkrankung, einer akuten Verletzung oder eines akuten Traumas, Schmerzen des oberen Rückens oder Schmerzen des unteren Rückens, zum Beispiel resultierend aus einer systemischen, regionalen oder primären Rückenmarkserkrankung, zum Beispiel Nervenwurzelerkrankung, Knochenschmerzen, zum Beispiel durch Osteoarthritis, Osteoporose, Knochenmetastase oder aus unbekannten Gründen, Beckenschmerzen, mit einer Rückenmarksverletzung assoziierten Schmerz, Herzbrustschmerz, Nichtherzbrustschmerz, zentralen Schmerz nach Schlaganfall, myofaszialen Schmerz, Krebsschmerz, AIDS-Schmerz, Sichelzellenschmerz, geriatrischen Schmerz oder Schmerz, verursacht durch Kopfweh, Kiefergelenksyndrom, Gicht, Fibrose oder Skalenus-Syndrom, Schmerz, bedingt durch Operation und Folgezustände der Operation.

8. Verwendung nach Anspruch 3, wobei das Schmerzsyndrom ein akuter Schmerz ist.

9. Verwendung nach Anspruch 8, wobei der akute Schmerz umfasst: akuten Schmerz, verursacht durch akute Verletzung, Krankheit, sportmedizinische Verletzungen, Karpaltunnelsyndrom, Verbrennungen, Verstauchungen und Zerrungen des Muskelskeletts, Muskel-Sehnen-Zerrung, cervicobrachiale Schmerzsyndrome, Magengeschwür, Duodenalulcus, Dysmenorrhoe, Endometriose oder Operation (zum Beispiel Operation am offenen Herzen oder Bypass-Operation), postoperativen Schmerz, Nierensteinschmerz, Gallenblasenschmerz, Gallensteinschmerz, Geburtsschmerz oder Zahnschmerz.

10. Verwendung nach Anspruch 3, wobei das Schmerzsyndrom vom inflammatorischen Typ ist.

11. Verwendung nach einem der Ansprüche 1 und 2, wobei die pathologische Affektion Migräne ist.

12. Verwendung nach Anspruch 11, wobei Migräne umfasst: transformierte Migräne oder sich entwickelndes Kopfweh, Cluster-Kopfweh, Spannungskopfschmerz sowie sekundäre Kopfschmerzstörungen wie zum Beispiel die, die von Infektionen, Stoffwechselstörungen oder anderen systemischen Erkrankungen herrühren, und anderen akuten Kopfschmerz, paroxysmale Migräne, resultierend aus einer Verschlechterung der oben genannten primären und sekundären Kopfschmerzen.

13. Verwendung nach Anspruch 1, wobei die pathologische Affektion ausgewählt ist aus Spondylarthritis, cervicaler Arthritis, Fibromyalgie, Gicht, juveniler rheumatoider Arthritis, lumbosakraler Arthritis, Osteoarthritis, Osteoporose, psoriatischer Arthritis und rheumatischer Erkrankung.

14. Verwendung nach Anspruch 1, wobei die pathologische Affektion aus Psoriasis, Dermatitis und Sonnenbrand ausgewählt ist.

15. Verwendung nach Anspruch 1, wobei die pathologische Affektion aus Asthma, allergischer Rhinitis, Atemnotsyndrom, Bronchitis und chronisch-obstruktiver Lungenerkrankung ausgewählt ist.

16. Verwendung nach Anspruch 1, wobei die pathologische Affektion aus Periarteritis nodosa, Schilddrüsenentzündung, Multipler Sklerose, Sarcoidose, Bechet-Syndrom, Polymyositis und Gingivitis ausgewählt ist.

17. Verwendung nach einem der Ansprüche 1 und 2, wobei die pathologische Affektion ausgewählt ist aus Colitis ulcerosa, Chron'scher Erkrankung, Ileitis, Proctitis, Zöliakie, Enteropathien, mikroskopischer oder kollagenöser Colitis, eosinophiler Gastroenteritis, Pouchitis, resultierend nach Proktokolektomie und nach ileonataler Anastomose, Reizdarmsyndrom, einschließlich Pylorospasmus, nervöser Verdauungsstörung, irritablem Colon, spastischer Colitis, Reizdarm, Intestinalneurose, funktioneller Colitis, mucomembranöser Colitis und laxierender Colitis, atrophischer Gastritis, Gastritis varialoforme, peptischem Ulcus, Pyrosis, Schädigung am GI-Trakt durch *Helicobacter pylori,* gastroösophagealer Refluxkrankheit, Gastroparese, zum Beispiel diabetische Gastroparese, Nicht-Ulcus-Dyspepsie (NUD), Emesis, Diarrhöe und visceraler Entzündung.

18. Verwendung nach einem der Ansprüche 1 und 2, wobei die pathologische Affektion ausgewählt ist aus hyperaktiver Blase, Prostatitis, zum Beispiel chronischer bakterieller und chronischer nichtbakterieller Prostatitis, Prostadynie, interstitieller Cystitis, Harninkontinenz, Adnexitis, Beckenentzündung, Bartholinitis und Vaginitis.

19. Verwendung von (R)-2-[4-(2-Fluorbenzyloxy)benzylamino]-propanamid ohne (S)-2-[4-(2-Fluorbenzyloxy)benzylamino]-propanamid oder eines pharmazeutisch verträglichen Salzes davon, vorzugsweise des Salzes mit Methansulfonsäure nach einem der Ansprüche 1 bis 18.

20. Verwendung von (R)-2-[4-(2-Chlorbenzyloxy)benzylamino]-N-methylpropanamid ohne (S)-2-[4-(2-Chlorbenzyloxy)benzylamino]-N-methylpropanamid oder eines pharmazeutisch verträglichen Salzes davon, vorzugsweise des Salzes mit Methansulfonsäure nach einem der Ansprüche 1 bis 18.

21. Verwendung nach einem der Ansprüche 1 bis 19, wobei das MAO-Enzym MAO-B-Isoform ist.

22. Verwendung von (R)-2-[4-(2-Fluorbenzyloxy)benzylamino]-propanamid ohne (S)-2-[4-(2-Fluorbenzyloxy)benzylamino]-propanamid oder (R)-2-[4-(2-Chlorbenzyloxy)benzylamino]-N-methylpropanamid ohne (S)-2-[4-(2-Chlorbenzyloxy)benzylamino]-N-methylpropanamid oder eines pharmazeutisch verträglichen Salzes davon, vorzugsweise des Salzes mit Methansulfonsäure, für die Herstellung eines Medikaments zur Prävention, Linderung und/oder Heilung eines Schmerzsyndroms, das bei den Dosierungen, die therapeutisch wirksam sind für die Behandlung des Schmerzsyndroms im Wesentlichen frei von einer MAO-B inhibierenden Wirkung ist oder eine deutlich reduzierte MAO-B inhibierende Wirkung aufweist.

23. Verwendung nach einem der Ansprüche 1 bis 22, die sowohl Behandlung von entwickelten Symptomen als auch eine prophylaktische Behandlung umfasst.

24. Verwendung nach einem der Ansprüche 1 bis 23, wobei das einzelne (R)-Isomer oder ein pharmazeutisch verträgliches Salz davon in Verbindung mit einem oder mehreren anderen therapeutischen Mitteln verwendet wird.

25. Verwendung nach Anspruch 24, wobei das andere therapeutische Mittel Gabapentin ist.

26. Pharmazeutische Zusammensetzung, die (R)-2-[4-(2-Fluorbenzyloxy)benzylamino]propanamid ohne (S)-2-[4-(2-Fluorbenzyloxy)benzylamino]propanamid oder ein pharmazeutisch verträgliches Salz davon, vorzugsweise sein Methansulfonat umfasst, zur Verwendung bei der selektiven Behandlung eines Schmerzsyndroms oder von Migräne, wobei die therapeutische Aktivität der Verbindung im Wesentlichen frei von einer MAO inhibierenden Nebenwirkung ist oder eine deutlich reduzierte MAO inhibierende Nebenwirkung aufweist.

27. Pharmazeutische Zusammensetzung nach Anspruch 26, wobei das Schmerzsyndrom entweder vom neuropathischen oder vom inflammatorischen Typ ist.

28. Pharmazeutische Zusammensetzung, die (R)-2-[4-(2-Chlorbenzyloxy)benzylamino]-N-methylpropanamid ohne (S)-2-[4-(2-Chlorbenzyloxy)benzylamino]-N-methylpropanamid oder ein pharmazeutisch verträgliches Salz davon, vorzugsweise sein Methansulfonat umfasst, zur Verwendung bei der selektiven Behandlung eines Schmerzsyndroms oder von Migräne, wobei die therapeutische Aktivität der Verbindung im Wesentlichen frei von einer MAO inhibierenden Nebenwirkung ist oder eine deutlich reduzierte MAO inhibierende Nebenwirkung aufweist.

29. Pharmazeutische Zusammensetzung nach Anspruch 28, wobei das Schmerzsyndrom entweder vom neuropathischen oder vom inflammatorischen Typ ist.

30. Verbindung, ausgewählt aus (R)-2-[4-(2-Fluorbenzyloxy)benzylamino]propanamid ohne (S)-2-[4-(2-Fluorbenzyloxy)benzylamino]propanamid und (R)-2-[4-(2-Chlorbenzyloxy)benzylamino]-N-methylpropanamid ohne (S)-2-[4-(2-Chlorbenzyloxy)benzylamino]-N-methylpropanamid, oder ein pharmazeutisch verträgliches Salz davon zur Verwendung als Medikament, das als Natrium- und/oder Calciumkanalmodulator selektiv aktiv ist, zur Verwendung bei der Prävention, Linderung und/oder Heilung einer pathologischen Affektion, ausgewählt aus Schmerz, Migräne, Spondylarthritis, cervikaler Arthritis, Fibromyalgie, Gicht, juveniler rheumatoider Arthritis, lumbosakraler Arthritis, Osteoarthritis, Osteoporose, psoriatischer Arthritis, rheumatischer Erkrankung, Psoriasis, Dermatitis, Sonnenbrand, Asthma, allergischer Rhinitis, Atemnotsyndrom, Bronchitis, chronisch-obstruktiver Lungenerkrankung, Periarteritis nodosa, Schilddrüsenentzündung, Multipler Sklerose, Sarcoidose, Bechet-Syndrom, Polymyositis, Gingivitis, Colitis ulcerosa, Chron'scher Erkrankung, Ileitis, Proctitis, Zöliakie, Enteropathien, mikroskopischer oder kollagenöser Colitis, eosinophiler Gastroenteritis, Pouchitis, resultierend nach Proktokolektomie und nach ileonataler Anastomose, Reizdarmsyndrom, einschließlich Pylorospasmus, nervöser Verdauungsstörung, irritablem Colon, spastischer Colitis, spastischem Darm, Intestinalneurose, Funktionscolitis, mucomembranöser Colitis und laxierender Colitis, atrophischer Gastritis, Gastritis varialoforme, peptischer Ulceration, Sodbrennen, Schädigung am GI-Trakt durch *Heliobacter pylori,* gastroösophagealer Refluxerkrankung, Gastroparese, zum Beispiel diabetischer Gastroparese, Nicht-Ulcus-Dyspepsie (NUD), Emesis, Diarrhöe, visceraler Entzündung, hyperaktiver Blase, Prostatitis, zum Beispiel chronischer bakterieller und chronischer nichtbakterieller Prostatitis, Prostadynie, interstitieller Cystitis, Harninkontinenz, Adnexitis, Beckenentzündung, Bartholinitis und Vaginitis, **dadurch gekennzeichnet, dass** das Medikament bei Dosierungen, die therapeutisch wirksam sind bei der Prävention, Linderung und/oder Heilung der pathologischen Affektion im Wesentlichen frei von einer MAO inhibierenden Wirkung ist oder eine deutlich reduzierte MAO inhibierende Wirkung hat.

31. Verbindung nach Anspruch 30 zur Verwendung nach Anspruch 30, wobei die pathologische Affektion ausgewählt ist aus einem Schmerzsyndrom, Migräne, Colitis ulcerosa, Chron'scher Erkrankung, Ileitis, Proctitis, Zöliakie, Enteropathien, mikroskopischer oder kollagenöser Colitis, eosinophiler Gastroenteritis, Pouchitis, resultierend nach Proktokolektomie und nach ileonataler Anastomose, Reizdarmsyndrom, einschließlich Pylorospasmus, nervöser Verdauungsstörung, irritablem Colon, spastischer Colitis, Reizdarm, intestinaler Neurose, funktioneller Colitis, mucomembranöser Colitis und laxierender Colitis, atrophischer Gastritis, Gastritis varialoforme, peptischer Ulceration, Pyrosis, Schädigung am GI-Trakt durch Heli*cobacter pylori,* gastroösophagealer Refluxerkrankung, Gastroparese, zum Beispiel diabetischer Gastroparese, Nicht-Ulcus-Dyspepsie (NUD), Emesis, Diarrhöe, visceraler Entzündung, hyperaktiver Blase, Prostatitis, zum Beispiel chronischer bakterieller und chronischer nichtbakterieller Prostatitis, Prostadynie, interstitieller Cystitis, Harninkontinenz, Adnexitis, Beckenentzündung, Bartholinitis und Vaginitis.

32. Verbindung nach Anspruch 30 zur Verwendung nach einem der Ansprüche 30 und 31, wobei die pathologische Affektion ein Schmerzsyndrom ist.

33. Verbindung nach Anspruch 30 zur Verwendung nach Anspruch 32, wobei das Schmerzsyndrom ein neuropathisches Schmerzsyndrom ist.

34. Verbindung nach Anspruch 30 zur Verwendung nach einem der Ansprüche 30 und 31, wobei die pathologische Affektion Migräne ist.

35. Verbindung nach Anspruch 30 zur Verwendung nach einem der Ansprüche 30 und 31, wobei die pathologische Affektion ein Schmerzsyndrom, wie in einem der Ansprüche 4 bis 10 definiert, ist.

36. Verbindung nach Anspruch 30 zur Verwendung nach einem der Ansprüche 30 und 31, wobei die pathologische Affektion Migräne, wie in Anspruch 12 definiert, ist.

## Revendications

1. Utilisation de (R)-2-[4-(2-fluorobenzyloxy)benzylamino]propanamide sans (S)-2-[4-(2-fluorobenzyloxy)benzylamino]propanamide ou de (R)-2-[4-(2-chlorobenzyloxy)benzylamino]-N-méthylpropanamide sans (S)-2-[4-(2-chlorobenzyloxy)benzylamino]-N-méthylpropanamide, ou d'un de leurs sels pharmaceutiquement acceptables, pour fabriquer un médicament agissant de manière sélective comme modulateur des canaux sodiques et/ou calciques pour prévenir, soulager et/ou guérir une affection pathologique choisie parmi les suivantes : douleur, migraine, spondylite ankylosante, arthrite cervicale, fibromyalgie, goutte, arthrite rhumatoïde juvénile, arthrite sacro-lombaire, ostéo-arthrite, ostéoporose, arthrite psoriasique, maladie rhumatismale, psoriasis, dermatite, coup de soleil, asthme, rhinite allergique, syndrome de détresse respiratoire, bronchite, bronchopneumopathie chronique obstructive, périartérite noueuse, thyroïdite, sclérose en plaques, sarcoïdose, syndrome de Behçet, polymyosite, gingivite, rectocolite hémorragique, iléite régionale, iléite, proctite, maladie coeliaque, entéropathies, colite microscopique ou collagène, gastro-entérite à éosinophiles, pochite consécutive à une proctocolectomie et à une anastomose iléo-anale, syndrome de l'intestin irritable y compris pylorospasme, indigestion nerveuse, spasme du côlon, colite spastique, spasme intestinal, névrose intestinale, colite fonctionnelle, colite muqueuse et colite aux laxatifs, gastrite atrophique, gastrite varioliforme, ulcère peptique, pyrosis, lésion gastro-intestinale due à *Helicobacter pylori,* reflux gastro-oesophagien, gastroparésie telle que la gastroparésie diabétique, dyspepsie non ulcéreuse (DNU), vomissement, diarrhée, inflammation viscérale, hyperactivité vésicale, prostatite telle que prostatite chronique bactérienne et non bactérienne, prostadynie, cystite interstitielle, incontinence urinaire, annexite, inflammation pelvienne, bartholinite et vaginite, **caractérisée en ce que** ledit médicament est pratiquement dénué d'effet d'inhibition de la MAO ou présente un effet d'inhibition de la MAO significativement réduit à des dosages qui sont thérapeutiquement efficaces pour prévenir, soulager et/ou guérir ladite affection pathologique.

2. Utilisation selon la revendication 1, dans laquelle l'affection pathologique est choisie parmi les suivantes : syndrome de douleur, migraine, rectocolite hémorragique, iléite régionale, iléite, proctite, maladie coeliaque, entéropathies, colite microscopique ou collagène, gastro-entérite à éosinophiles, pochite consécutive à une proctocolectomie et à une anastomose iléo-anale, syndrome de l'intestin irritable y compris pylorospasme, indigestion nerveuse, spasme du côlon, colite spastique, spasme intestinal, névrose intestinale, colite fonctionnelle, colite muqueuse et colite aux laxatifs, gastrite atrophique, gastrite varioliforme, ulcère peptique, pyrosis, lésion gastro-intestinale due à *Helicobacter pylori,* reflux gastro-oesophagien, gastroparésie telle que la gastroparésie diabétique, dyspepsie non ulcéreuse (DNU), vomissement, diarrhée, inflammation viscérale, hyperactivité vésicale, prostatite telle que prostatite chronique bactérienne et non bactérienne, prostadynie, cystite interstitielle, incontinence urinaire, annexite, inflammation pelvienne, bartholinite et vaginite.

3. Utilisation selon l'une quelconque des revendications 1 et 2, dans laquelle l'affection pathologique est un syndrome de douleur.

4. Utilisation selon la revendication 3, dans laquelle le syndrome de douleur est un syndrome de douleur neuropathique.

5. Utilisation selon la revendication 4, dans laquelle le syndrome de douleur neuropathique comprend les troubles suivants : neuropathie diabétique, sciatique, douleur non spécifique dans le bas du dos, douleur liée à la sclérose en plaques, fibromyalgie, neuropathie liée au VIH, névralgie telle que névralgie post-herpétique et névralgie trigéminale, et douleur résultant de traumatisme physique, amputation, cancer, toxines ou états inflammatoires chroniques, de compressions de la moelle épinière, de racines nerveuses, de nerfs périphériques et des voies nociceptives centrales.

6. Utilisation selon la revendication 3, dans laquelle le syndrome de douleur est une douleur chronique.

7. Utilisation selon la revendication 6, dans laquelle la douleur chronique comprend une douleur chronique due à une inflammation, ostéoarthrite, arthrite rhumatoïde ou séquelle de la maladie, lésion aiguë ou traumatisme, douleur du haut du dos ou douleur du bas du dos résultant par exemple d'une pathologie systémique, locale ou d'une affection rachidienne primaire telle qu'une radiculopathie, douleur osseuse due par exemple à une ostéoarthrite, une ostéoporose, des métastases osseuses ou des causes inconnues, douleur pelvienne, douleur associée à une lésion de la moelle épinière, douleur thoracique d'origine cardiaque, douleur thoracique non cardiaque, douleur centrale consécutive à un AVC, douleur myofasciale, douleur liée à un cancer, douleur liée au SIDA, douleur liée à l'anémie falciforme, douleur gériatrique ou douleur provoquée par une céphalée, le syndrome algo-dysfonctionnel de l'appareil manducateur, la goutte, une fibrose ou le syndrome du défilé thoracique, douleur liée à la chirurgie et aux séquelles de la chirurgie.

8. Utilisation selon la revendication 3, dans laquelle le syndrome de douleur est une douleur aiguë.

9. Utilisation selon la revendication 8, dans laquelle la douleur aiguë comprend une douleur aiguë provoquée par : lésion aiguë, maladie, lésion de médecine sportive, syndrome du tunnel carpien, brûlure, distensions et claquages musculo-squelettiques, claquage musculo-tendineux, syndromes de douleur cervico-brachiale, ulcère gastrique, ulcère duodénal, dysménorrhée, endométriose ou chirurgie (par exemple opération à coeur ouvert ou pontage), douleur post-opératoire, douleur due à des calculs rénaux, douleur de la vésicule biliaire, douleur due à des calculs biliaires, douleur obstétrique ou douleur dentaire.

10. Utilisation selon la revendication 3, dans laquelle le syndrome de douleur est de type inflammatoire.

11. Utilisation selon l'une quelconque des revendications 1 et 2, dans laquelle l'affection pathologique est la migraine.

12. Utilisation selon la revendication 11, dans laquelle la migraine comprend : migraine transformée ou céphalée évolutive, algie vasculaire de la face, céphalée de tension ainsi que troubles céphaleux secondaires tels que ceux découlant d'infections, troubles métaboliques ou autres maladies systémiques et autres céphalées aiguës, hémicrânie paroxystique résultant d'une aggravation des céphalées primaires et secondaires précitées.

13. Utilisation selon la revendication 1, dans laquelle l'affection pathologique est choisie parmi les suivantes : spondylite ankylosante, arthrite cervicale, fibromyalgie, goutte, arthrite rhumatoïde juvénile, arthrite sacro-lombaire, ostéoarthrite, ostéoporose, arthrite psoriasique et maladie rhumatismale.

14. Utilisation selon la revendication 1, dans laquelle l'affection pathologique est choisie parmi les suivantes : psoriasis, dermatite et coup de soleil.

15. Utilisation selon la revendication 1, dans laquelle l'affection pathologique est choisie parmi les suivantes : asthme, rhinite allergique, syndrome de détresse respiratoire, bronchite et bronchopneumopathie chronique obstructive.

16. Utilisation selon la revendication 1, dans laquelle l'affection pathologique est choisie parmi les suivantes : périartérite noueuse, thyroïdite, sclérose en plaques, sarcoïdose, syndrome de Behçet, polymyosite et gingivite.

17. Utilisation selon l'une quelconque des revendications 1 et 2, dans laquelle l'affection pathologique est choisie parmi les suivantes : rectocolite hémorragique, iléite régionale, iléite, proctite, maladie coeliaque, entéropathies, colite microscopique ou collagène, gastro-entérite à éosinophiles, pochite consécutive à une proctocolectomie et à une anastomose iléo-anale, syndrome de l'intestin irritable y compris pylorospasme, indigestion nerveuse, spasme du côlon, colite spastique, spasme intestinal, névrose intestinale, colite fonctionnelle, colite muqueuse et colite aux laxatifs, gastrite atrophique, gastrite varioliforme, ulcère peptique, pyrosis, lésion gastro-intestinale due à *Helicobacter pylori,* reflux gastro-oesophagien, gastroparésie telle que la gastroparésie diabétique, dyspepsie non ulcéreuse (DNU), vomissement, diarrhée et inflammation viscérale.

18. Utilisation selon l'une quelconque des revendications 1 et 2, dans laquelle l'affection pathologique est choisie parmi les suivantes : hyperactivité vésicale, prostatite telle que prostatite chronique bactérienne et non bactérienne, prostadynie, cystite interstitielle, incontinence urinaire, annexite, inflammation pelvienne, bartholinite et vaginite.

19. Utilisation de (R)-2-[4-(2-fluorobenzyloxy)benzylamino]-propanamide sans (S)-2-[4-(2-fluorobenzyloxy)benzylamino]propanamide, ou d'un de ses sels pharmaceutiquement acceptables, de préférence le sel de l'acide méthanesulfonique, selon l'une quelconque des revendications 1 à 18.

20. Utilisation de (R)-2-[4-(2-chlorobenzyloxy)benzylamino]-N-méthylpropanamide sans (S)-2-[4-(2-chlorobenzyloxy)benzylamino]-N-méthylpropanamide, ou d'un de ses sels pharmaceutiquement acceptables, de préférence le sel de l'acide méthanesulfonique, selon l'une quelconque des revendications 1 à 18.

21. Utilisation selon l'une quelconque des revendications 1 à 19, dans laquelle l'enzyme MAO est l'isoforme MAO-B.

22. Utilisation de (R)-2-[4-(2-fluorobenzyloxy)benzylamino]-propanamide sans (S)-2-[4-(2-fluorobenzyloxy)benzylamino]propanamide ou de (R)-2-[4-(2-chlorobenzyloxy)benzylamino]-N-méthylpropanamide sans (S)-2-[4-(2-chlorobenzyloxy)benzylamino]-N-méthylpropanamide, ou d'un de leurs sels pharmaceutiquement acceptables, de préférence le sel de l'acide méthanesulfonique, pour préparer un médicament destiné à prévenir, soulager et/ou guérir un syndrome de douleur et qui, aux dosages qui sont thérapeutiquement efficaces pour le traitement dudit syndrome de douleur, est pratiquement dénué d'effet d'inhibition de la MAO-B ou présente un effet d'inhibition de la MAO-B significativement réduit.

23. Utilisation selon l'une quelconque des revendications 1 à 22, comprenant à la fois le traitement de symptômes déclarés et le traitement prophylactique.

24. Utilisation selon l'une quelconque des revendications 1 à 23, dans laquelle l'isomère (R) seul ou un de ses sels pharmaceutiquement acceptables est utilisé en combinaison avec un ou plusieurs autres agents thérapeutiques.

25. Utilisation selon la revendication 24, dans laquelle l'autre agent thérapeutique est la gabapentine.

26. Composition pharmaceutique comprenant du (R)-2-[4-(2-fluorobenzyloxy)benzylamino]propanamide sans (S)-2-[4-(2-fluorobenzyloxy)benzylamino]propanamide, ou un de ses sels pharmaceutiquement acceptables, de préférence son méthanesulfonate, destinée à être utilisée dans le traitement sélectif d'un syndrome de douleur ou de la migraine, dans laquelle l'activité thérapeutique dudit composé est pratiquement dénuée d'effet secondaire d'inhibition de la MAO ou présente un effet secondaire d'inhibition de la MAO significativement réduit.

27. Composition pharmaceutique selon la revendication 26, dans laquelle le syndrome de douleur est de type soit neuropathique, soit inflammatoire.

28. Composition pharmaceutique comprenant du (R)-2-[4-(2-chlorobenzyloxy)benzylamino]-N-méthylpropanamide sans (S)-2-[4-(2-chlorobenzyloxy)benzylamino]-N-méthylpropanamide, ou un de ses sels pharmaceutiquement acceptables, de préférence son méthanesulfonate, destinée à être utilisée dans le traitement sélectif d'un syndrome de douleur ou de la migraine, dans laquelle l'activité thérapeutique dudit composé est pratiquement dénuée d'effet secondaire d'inhibition de la MAO ou présente un effet secondaire d'inhibition de la MAO significativement réduit.

29. Composition pharmaceutique selon la revendication 28, dans laquelle le syndrome de douleur est de type soit neuropathique, soit inflammatoire.

30. Composé choisi parmi le (R)-2-[4-(2-fluorobenzyloxy)-benzylamino]propanamide sans (S)-2-[4-(2-fluorobenzyloxy)benzylamino]propanamide et le (R)-2-(4-(2-chlorobenzyloxy)benzylamino]-N-méthylpropanamide sans (S)-2-[4-chlorobenzyloxy)benzylamino)-N-méthylpropanamide, ou un de leurs sels pharmaceutiquement acceptables, destiné à être utilisé comme un médicament agissant de manière sélective comme modulateur des canaux sodiques et/ou calciques et destiné à être utilisé pour prévenir, soulager et/ou guérir une affection pathologique choisie parmi les suivantes : douleur, migraine, spondylite ankylosante, arthrite cervicale, fibromyalgie, goutte, arthrite rhumatoïde juvénile, arthrite sacro-lombaire, ostéoarthrite, ostéoporose, arthrite psoriasique, maladie rhumatismale, psoriasis, dermatite, coup de soleil, asthme, rhinite allergique, syndrome de détresse respiratoire, bronchite, bronchopneumopathie chronique obstructive, périartérite noueuse, thyroïdite, sclérose en plaques, sarcoïdose, syndrome de Behçet, polymyosite, gingivite, rectocolite hémorragique, iléite régionale, iléite, proctite, maladie coeliaque, entéropathies, colite microscopique ou collagène, gastro-entérite à éosinophiles, pochite consécutive à une proctocolectomie et à une anastomose iléo-anale, syndrome de l'intestin irritable y compris pylorospasme, indigestion nerveuse, spasme du côlon, colite spastique, spasme intestinal, névrose intestinale, colite fonctionnelle, colite muqueuse et colite aux laxatifs, gastrite atrophique, gastrite varioliforme, ulcère peptique, pyrosis, lésion gastro-intestinale due à *Helicobacter pylori,* reflux gastro-oesophagien, gastroparésie telle que la gastroparésie diabétique, dyspepsie non ulcéreuse (DNU), vomissement, diarrhée, inflammation viscérale, hyperactivité vésicale, prostatite telle que prostatite chronique bactérienne et non bactérienne, prostadynie, cystite interstitielle, incontinence urinaire, annexite, inflammation pelvienne, bartholinite et vaginite, **caractérisé en ce que** ledit médicament est pratiquement dénué d'effet d'inhibition de la MAO ou présente un effet d'inhibition de la MAO significativement réduit aux dosages qui sont thérapeutiquement efficaces pour prévenir, soulager et/ou guérir ladite affection pathologique.

31. Composé selon la revendication 30 pour une utilisation selon la revendication 30, dans laquelle l'affection pathologique est choisie parmi les suivantes : syndrome de douleur, migraine, rectocolite hémorragique, iléite régionale, iléite, proctite, maladie coeliaque, entéropathies, colite microscopique ou collagène, gastro-entérite à éosinophiles, pochite consécutive à une proctocolectomie et à une anastomose iléo-anale, syndrome de l'intestin irritable y compris pylorospasme, indigestion nerveuse, spasme du côlon, colite spastique, spasme intestinal, névrose intestinale, colite fonctionnelle, colite muqueuse et colite aux laxatifs, gastrite atrophique, gastrite varioliforme, ulcère peptique, pyrosis, lésion gastro-intestinale due à *Helicobacter pylori,* reflux gastro-oesophagien, gastroparésie telle que la gastroparésie diabétique, dyspepsie non ulcéreuse (DNU), vomissement, diarrhée, inflammation viscérale, hyperactivité vésicale, prostatite telle que prostatite chronique bactérienne et non bactérienne, prostadynie, cystite interstitielle, incontinence urinaire, annexite, inflammation pelvienne, bartholinite et vaginite.

32. Composé selon la revendication 30 pour une utilisation selon l'une quelconque des revendications 30 et 31, dans laquelle l'affection pathologique est un syndrome de douleur.

33. Composé selon la revendication 30 pour une utilisation selon la revendication 32, dans laquelle le syndrome de douleur est un syndrome de douleur neuropathique.

34. Composé selon la revendication 30 pour une utilisation selon l'une quelconque des revendications 30 et 31, dans laquelle l'affection pathologique est la migraine.

35. Composé selon la revendication 30 pour une utilisation selon l'une quelconque des revendications 30 et 31, dans laquelle l'affection pathologique est un syndrome de douleur tel que défini dans l'une quelconque des revendications 4 à 10.

36. Composé selon la revendication 30 pour une utilisation selon l'une quelconque des revendications 30 et 31, dans laquelle l'affection pathologique est la migraine telle que définie dans la revendication 12.
